# EUROPEAN PATENT APPLICATION

(11) **EP 3 943 606 A1**
(43) Date of publication of application: **26.01.2022**
(21) Application number: 20774391.5
(22) Date of filing: 18.03.2020
(51) Int. Cl.: C12P 13/06, C07C 227/18, C07C 231/24, C07C 231/02

(54) **METHODS FOR PREPARING B-ALANINE, B-ALANINE SALT AND PANTOTHENATE**

(30) Priority: 20.03.2019 CN 201910212635; 22.01.2020 CN 202010075180
(71) Applicant: Guang an Mojia Biotechnology Co., Ltd., Guang An, Sichuan 638000 (CN)
(72) Inventor: LAU, Man Kit, Sichuan 638000 (CN); CHEN, Yan, Sichuan 638000 (CN); CHIEW, Ansen, Sichuan 638000 (CN); LU, Chengliang, Sichuan 638000 (CN); ZENG, Congming, Sichuan 638000 (CN); SU, Jinhuan, Sichuan 638000 (CN); LU, Weihua, Sichuan 638000 (CN)
(74) Representative: Straus, Alexander
(86) International application number: PCT/CN2020/079957
(87) International publication number: WO 2020/187256

(57) **Abstract**

Provided is a method for preparing β-alanine, the method comprising: preparing a β-alanine product from a reactant containing fumaric acid and aqueous ammonia in the presence of a catalyst, wherein the catalyst contains a catalyst composition containing aspartase and L-aspartic acid-α-decarboxylase, and adding fumaric acid during the reaction, wherein the total moles of the fumaric acid added is equal to the initial moles of the aqueous ammonia in the reactant minus the initial moles of the fumaric acid in the reactant. Also provided are methods for preparing a β-alanine salt (in particular calcium β-alanine, sodium β-alanine, and potassium β-alanine) and a pantothenate (in particular calcium pantothenate, sodium pantothenate, and potassium pantothenate).

## Description

### FIELD OF THE INVENTION

The invention belongs to biotechnology field, and specifically relates to a method for enzymatic preparation of β-alanine, β-alanine salt (in particular calcium β-alanine, sodium β-alanine, and potassium β-alanine), and a pantothenate (in particular calcium pantothenate, sodium pantothenate, and potassium pantothenate).

### RELATED ART

β-alanine, also known as β-aminopropionic acid or 3-aminopropionic acid, is a β-type non-protein amino acid found in nature. β-alanine is a multi-purpose organic synthetic raw material, mainly used to synthesize pantothenic acid and calcium pantothenate, carnosine, pamidronate, balsalazide, etc., which is widely used in medicine, feed, food and other fields, and has a very large market demand.

At present, the methods for producing β-alanine are divided into two categories: chemical synthesis and biological methods. Due to former researching and the mature process in the synthesis of β-alanine, the chemical method is still the main production method used at the domestic and abroad.

There are two main chemical methods to produce β-alanine. One involves reacting acrylic acid with ammonia at a high temperature and a high pressure. This method has a low cost, but has high requirements for the safety due to high corrosion on the equipment caused by strong acidity. The other involves firstly reacting acrylonitrile with ammonia at a high temperature and a high pressure to prepare β-aminopropionitrile, and then hydrolyzing the β-aminopropionitrile with sodium hydroxide under high temperature conditions. This method has a low yield, and a large amount of inorganic salts generated during the reaction results in a separation problem.

The chemical methods generally include harsh reaction conditions, require high qualities for equipment, cause environmental pollution, and have other problems. Therefore, with the continuous increasing use of β-alanine, biological methods have gradually become research hotspots due to their advantages in mild reaction conditions, high efficiency and environmental friendliness.

Production of β-alanine by the biological methods mostly uses microorganisms capable of producing specific enzymes in transforming substrates into β-alanine. For example, an aminating enzyme was used by Zhejiang University of Technology (CN1285730) to synthesize β-alanine from acrylic acid and ammonia. The method has high reaction efficiency and low cost, but there have been no reports on its application in industry at present due to the strong corrosion and irritation of the raw materials. Organonitrile hydrolase was used by Toshihiro Oikawa et al. (JP 10-42886) to catalyze β-aminopropionitrile to synthesize β-alanine. This method has high price in the raw materials and low reaction concentration: it has high cost, and is difficult to meet the requirements of industrial production.

Another biological method for synthesizing β-alanine involves using L-aspartate-α-decarboxylase to specifically remove α-carboxyl group in L-aspartic acid to produce β-alanine. However, the existing methods have the problems of low enzyme activity and poor enzyme stability.

### SUMMARY

In one aspect, the present invention provides a method for preparing β-alanine, comprising: preparing a β-alanine product from a reactant containing fumaric acid and aqueous ammonia in the presence of a catalyst, wherein the catalyst comprises a catalytic composition containing aspartase and L-aspartate-α-decarboxylase, and adding fumaric acid during the reaction, wherein the total moles of the fumaric acid added is equal to the initial moles of the aqueous ammonia in the reactant minus the initial moles of the fumaric acid in the reactant.

In some embodiments, the catalytic composition contains purified aspartase and purified L-aspartate-α-decarboxylase. In some embodiments, the catalytic composition contains bacteria expressing aspartase and L-aspartate-α-decarboxylase. In some embodiments, the bacteria comprise wet bacteria, immobilized bacteria, or disrupted liquid of bacteria. In some embodiments, the bacteria are derived from recombinantly engineered bacteria. In some embodiments, the bacteria comprise bacteria expressing aspartase alone and bacteria expressing L-aspartate-α-decarboxylase alone. In some embodiments, the weight percentage of the bacteria expressing aspartase alone to the initial fumaric acid in the reactant is 0.5%-4% (w/w); and the weight percentage of the bacteria expressing L-aspartate-α-decarboxylase alone to the initial fumaric acid in the reactant is 10%-30% (w/w). In some embodiments, the bacteria comprise bacteria co-expressing aspartase and L-aspartate-α-decarboxylase. In some embodiments, the weight percentage of the bacteria co-expressing aspartase and L-aspartate-α-decarboxylase to the initial fumaric acid in the reactant is 10%-40% (w/w). In some embodiments, the aspartase is derived from *Anoxybacillus flavithermus* or *Geobacillus thermodenitrificans;* the L-aspartate-α-decarboxylase is derived from *Bacillus thermotolerans, Anoxybacillus flavithermus* or *Methanocaldococcus jannaschii.* In some embodiments, the aspartase is derived from *Anoxybacillusflavithermus* WK1 or *Geobacillus thermodenitrificans* NG80-2; the L-aspartate-α-decarboxylase is derived from *Quasibacillus thermotolerans, Anoxybacillus flavithermus* AK1 or *Methanocaldococcus jannaschii* DSM 2661.

In some embodiments, the initial molar ratio of the fumaric acid to the aqueous ammonia in the reactant is 1:2. In some embodiments, the added fumaric acid is added in a fed-batch manner during the reaction. In some embodiments, the concentration of fumaric acid is ranged from 50 to 400 g/L, and the fed-batch speed thereof allows the pH value to be controlled at 6.8-7.2 during the reaction. In some embodiments, the reaction temperature is controlled at 25°C-55°C.

In some embodiments, the method for preparing β-alanine according to the present invention further comprises removing residues in the catalytic composition after the catalytic reaction is completed. In some embodiments, the method for preparing β-alanine according to the present invention further comprises crystallizing the β-alanine product. In some embodiments, a mother liquor is obtained after the crystallization, and the content of inorganic salts in the mother liquor is less than 10 g/L. In some embodiments, the mother liquor obtained after the crystallization can be recycled. In some embodiments, β-alanine crystal is obtained after the crystallization, and the content of inorganic salts in the crystal is less than 20 mg/g.

In another aspect, the present invention provides a method for preparing β-alanine salt, comprising the following steps of:
(a) preparing β-alanine by the method according to the present invention; and
(b) reacting the β-alanine obtained in step (a) with an alkaline solution.

In some embodiments, the β-alanine salt is an alkali metal or alkaline earth metal salt of β-alanine, and the alkaline solution is one containing alkali metal or alkaline earth metal cations. In some embodiments, the alkaline solution is selected from the group consisting of KOH, NaOH, Ca(OH)₂, Mg(OH)₂, Al(OH)₃, or a combination thereof. In some embodiments, the Ca(OH)₂ is obtained from the reaction of calcium oxide with water. In some embodiments, the β-alanine salt is calcium β-alanine, potassium β-alanine or sodium β-alanine. In some embodiments, the β-alanine salt is calcium β-alanine. In some embodiments, the method for preparing the β-alanine salt according to the present invention further comprises crystallizing the β-alanine salt.

In another aspect, the present invention provides a method for preparing pantothenate, comprising the following steps of:
(a) preparing β-alanine by the method according to the present invention;
(b) reacting the β-alanine obtained in step (a) with an alkaline solution to prepare β-alanine salt; and
(c) reacting pantolactone or pantoic acid with the β-alanine salt obtained in step (b).

In some embodiments, the pantolactone is D-pantolactone. In some embodiments, the pantoic acid is D-pantoic acid. In some embodiments, pantolactone or pantoic acid is dissolved in a solvent before reacting the pantolactone (for example D-pantolactone) or pantoic acid (for example D-pantoic acid) with the β-alanine salt obtained in step (b). In some embodiments, the solvent is methanol or ethanol. In some embodiments, the pantothenate is calcium pantothenate, sodium pantothenate or potassium pantothenate. In some embodiments, the pantothenate is calcium pantothenate. In some embodiments, the method for preparing pantothenate according to the present invention further comprises crystallizing the pantothenate.

In another aspect, the present invention provides a calcium pantothenate, wherein the content of chloride ion (by weight) is not higher than 190 ppm, and/or the content of a sodium ion (by weight) is not higher than 2200 ppm. In some embodiments, the calcium pantothenate is obtained by the method for preparing calcium pantothenate according to the present invention.

### BRIEF DESCRIPTION OF THE DRAWINGS

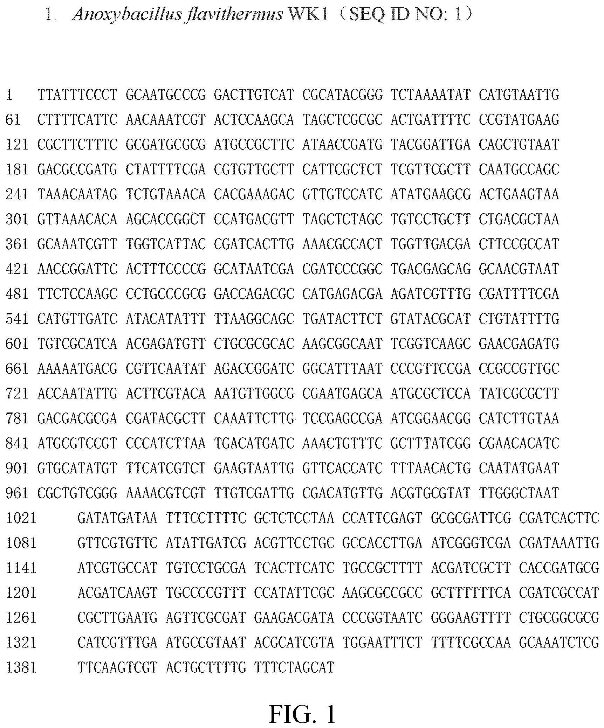

FIG. 1: shows SEQ ID NO: 1, the gene sequence encoding aspartase in the genome sequence of *Anoxybacillus flavithermus* WK1.

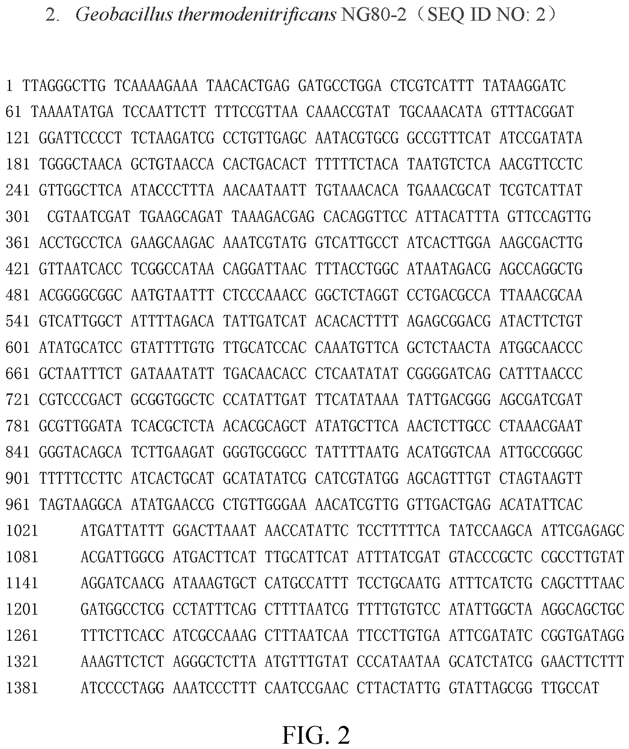

FIG. 2: shows SEQ ID NO:2, the gene sequence encoding aspartase in the genome sequence of *Geobacillus thermodenitrificans* NG80-2.

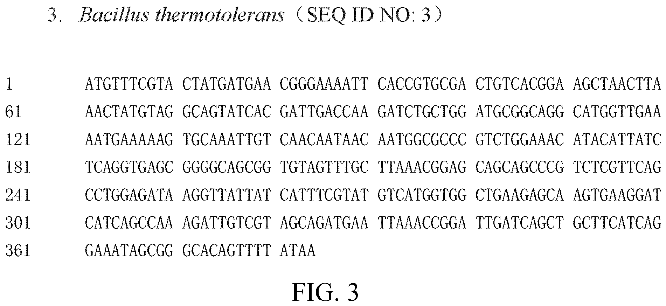

FIG. 3: shows SEQ ID NO:3, the gene sequence encoding L-aspartate-α-decarboxylase in the genome sequence of *Quasibacillus thermotolerans strain SGZ-8 Contig4* in *Bacillus thermotolerans.*

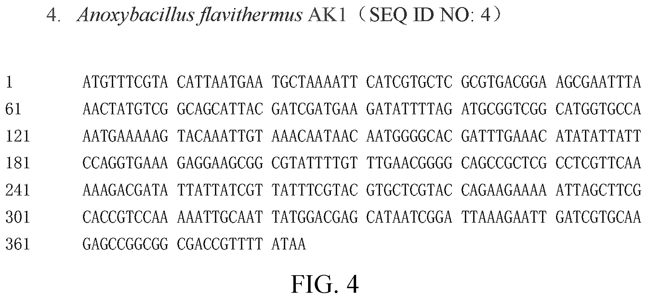

FIG. 4: shows SEQ ID NO:4, the gene sequence encoding L-aspartate-α-decarboxylase in the genome sequence of *Anoxybacillusflavithermus* AK1.

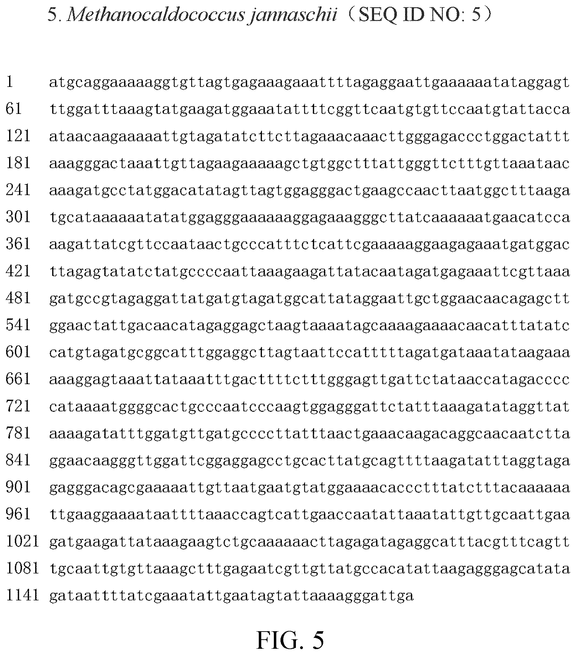

FIG. 5: shows SEQ ID NO:5, the gene sequence encoding L-aspartate-α-decarboxylase in the genome sequence of *Methanocaldococcus jannaschii.*
FIG. 6: shows an HPLC chromatogram demonstrating the linear relationship of the test method in the experiment for detecting the chloride ion and sodium ion content of calcium pantothenate. The figure shows that the retention time of chloride ions in the chromatographic column is 4.024 minutes, and the retention time of sodium ions in the chromatographic column is 4.351 minutes.
FIG. 7: shows a standard curve (FIG. 7A) plotted with the logarithm of the injection concentration of chloride ion (LgC) versus the logarithm of the peak area (LgA), and a standard curve (FIG. 7B) plotted with the logarithm of the injection concentration of sodium ion (LgC) versus the logarithm of the peak area (LgA) in the experiment for detecting the chloride ion and sodium ion content of calcium pantothenate.
FIG. 8: shows an HPLC chromatogram illustrating the detection limit result of chloride ion in the experiment for detecting the chloride ion and sodium ion content of calcium pantothenate.
FIG. 9: shows an HPLC chromatogram illustrating the quantification limit result of chloride ion in the experiment for detecting the chloride ion and sodium ion content of calcium pantothenate.
FIG. 10: shows an HPLC chromatogram of testing sample 1, testing sample 2, and testing sample 3 of calcium pantothenate in the experiment for detecting the chloride ion and sodium ion content of calcium pantothenate.

### DETAILED DESCRIPTION

The present invention overcomes the shortcomings of the existing β-alanine preparation process, and provides a green, high-efficiency and low-cost production process suitable for industrially producing β-alanine, β-alanine salt and pantothenate.

In one aspect, the present invention provides a method for preparing β-alanine, comprising: preparing a β-alanine product from a reactant containing fumaric acid and aqueous ammonia in the presence of a catalyst, wherein the catalyst includes a catalytic composition containing aspartase and L-aspartate-α-decarboxylase, and adding fumaric acid during the reaction, wherein the total moles of the fumaric acid added is equal to the initial moles of the aqueous ammonia in the reactant minus the initial moles of the fumaric acid in the reactant.

The "initial moles" or "initial mole" of fumaric acid or aqueous ammonia refer to the initial moles of fumaric acid or aqueous ammonia before the start of the catalytic reaction. The weight of "initial fumaric acid" in the reactant refers to the initial weight of fumaric acid added to the reactant before the start of the catalytic reaction. In some embodiments, the initial molar ratio of the aqueous ammonia to the fumaric acid in the reactant is 2:1, or fluctuates within the range of 10%, 9%, 8%, 7%, 6%, 5%, 4%, 3%, 2%, 1% above or below this value.

In some embodiments, the reactant containing fumaric acid and aqueous ammonia may contain ammonium fumarate. Ammonium fumarate refers to the product obtained by acid-base neutralization between fumaric acid and aqueous ammonia. Without being limited by theory, it is believed that in the reactant containing fumaric acid and aqueous ammonia, at least part of the fumaric acid and part of the aqueous ammonia will spontaneously form ammonium fumarate. In the present application, it is believed that 1 mole of ammonium fumarate is equivalent to 2 moles of aqueous ammonia and 1 mole of fumaric acid. Therefore, when the reactant contains only 1 mole of ammonium fumarate, it is believed that the ratio of the initial moles of aqueous ammonia to fumaric acid therein is 2:1.

In the method provided in the present application, the catalyst comprises a catalytic composition containing aspartase and L-aspartate-α-decarboxylase. Any known aspartase and L-aspartate-α-decarboxylase can be used. It is well known in the art that aspartase and L-aspartate-α-decarboxylase are known to be naturally expressed in a variety of microorganisms, and both have corresponding catalytic activities.

In some embodiments, the aspartase and L-aspartate-α-decarboxylase are derived from bacteria. In some embodiments, the aspartase is derived from *Anoxybacillus flavithermus* or *Geobacillus thermodenitrificans.* In some embodiments, the aspartase is derived from *Anoxybacillus flavithermus* WK1 strain or *Geobacillus thermodenitrificans* NG80-2 strain. In some embodiments, the amino acid sequence of the aspartase is the same as the amino acid sequence encoded by SEQ ID NO: 1 or SEQ ID NO:2, or has at least 85%, 86%, 87%, 88%, 89%, 90%, 91%, 92%, 93%, 94%, 95%, 96%, 97%, 98%, or 99% homology therewith.

In some embodiments, the L-aspartate-α-decarboxylase is derived from *Bacillus thermotolerans, Anoxybacillus flavithermus* or *Methanocaldococcus jannaschii.* In some embodiments, the L-aspartate-α-decarboxylase is derived from *Quasibacillus thermotolerans, Anoxybacillus flavithermus* AK1 or *Methanocaldococcus jannaschii* DSM 2661. In some embodiments, the amino acid sequence of the L-aspartate-α-decarboxylase is the same as the amino acid sequence encoded by SEQ ID NO:3, SEQ ID NO:4, or SEQ ID NO:5, or has at least 85%, 86%, 87%, 88%, 89%, 90%, 91%, 92%, 93%, 94%, 95%, 96%, 97%, 98%, or 99% homology therewith.

"Homology" in the present application involves: as to animo acid sequence, performing the sequence alignment between the candidate amino acid sequence and the reference amino acid sequence, introducing gaps when necessary to maximize the number of identical amino acids, and calculating percentage of the identical amino acids between the two amino acid sequences on this basis in terms of amino acid sequences; as to nucleic sequence, performing the sequence alignment between the candidate nucleic acid sequence and the reference nucleic acid sequence, introducing gaps when necessary to maximize the number of identical nucleotides, and calculating percentage of the identical nucleotides between the two nucleic acid sequences on this basis in terms of nucleic acid sequences. In other words, the percentage of homology between two amino acid sequences (or nucleotide sequences) can be calculated as follows: dividing the number of amino acids (or nucleotides) that are the same as the amino acids (or nucleotides) in the aligned reference sequence by the total number of amino acids (or nucleotides) in the candidate sequence or the reference sequence (the shorter prevails). The percentage of homology can be determined by aligning in a variety of ways known in the art. For example, the following publicly available tools can be used for sequence alignment, such as BLASTp (National Center for Biotechnology Information (NCBI): http://blast.ncbi.nlm.nih.gov/Blast.cgi, or see Altschul S.F. et al., J. Mol. Biol., 215:403-410 (1990); Stephen F. et al., Nucleic Acids Res., 25:3389-3402 (1997)) and ClustalW2 (European Biological Information Institute website: http://www.ebi.ac.uk/Tools/msa/clustalw2/, see Higgins D.G. et al., Methods in Enzymology, 266:383-402 (1996); Larkin M.A. et al., Bioinformatics (Oxford, England), 23(21): 2947-8 (2007)). When using software for sequence alignment, the default parameters provided by the software can be applied, or the parameters can be adjusted appropriately according to the needs of the alignment, and both are within the knowledge of those skilled in the art.

In the present application, the aspartase and L-aspartate-α-decarboxylase in the catalytic composition can exist in any suitable active form, such as but not limited to, isolated or purified active enzyme protein, natural or recombinant cells expressing the enzyme or lysates thereof.

In some embodiments, the catalytic composition contains purified aspartase and purified L-aspartate-α-decarboxylase. The purified enzyme may be obtained by isolation and purification from microorganisms that naturally express the enzyme, or may be obtained by recombinant expression followed by isolation and purification. Those skilled in the art can use conventional technical means in the art to prepare purified aspartase and L-aspartate-α-decarboxylase. For example, after ammonium sulfate precipitation, ion exchange chromatography is followed by gel chromatography.

In some embodiments, the catalytic composition contains bacteria expressing aspartase and L-aspartate-α-decarboxylase. In some embodiments, the bacteria comprise wet bacteria, immobilized bacteria, or disrupted liquid of bacteria. In some embodiments, the wet bacteria are obtained after the solid-liquid separation of the bacterial culture fermentation liquid, such as the bacteria collected by centrifugation; the immobilized bacteria are obtained by treating the wet bacteria via a conventional immobilization means, such as the bacteria embedded in sodium alginate; the disrupted liquid of bacteria is the solution obtained by conventionally disrupting the bacteria, such as a high-pressure homogenized disrupted liquid. The disrupted liquid of bacteria contains the required enzymes.

In some embodiments, the bacteria expressing aspartase and L-aspartate-α-decarboxylase are derived from wild-type bacteria. For example, wild-type bacteria that naturally express aspartase, wild-type bacteria that naturally express L-aspartate-α-decarboxylase, or wild-type bacteria that naturally express both aspartase and L-aspartate-α-decarboxylase can be used. The wild-type bacteria include, for example, *Anoxybacillus flavithermus, Geobacillus thermodenitrificans, Bacillus thermotolerans* and *Methanocaldococcus jannaschii.*

In some embodiments, the bacteria expressing aspartase and L-aspartate-α-decarboxylase are derived from recombinantly engineered bacteria. The recombinant engineered bacteria refer to the engineered bacteria that have introduced foreign genes into host engineered bacteria through recombinant DNA methods. Recombinant engineered bacteria can recombinantly express introduced foreign genes. Those skilled in the art can select a suitable host to express aspartase and L-aspartate-α-decarboxylase according to their actual needs. In some embodiments, the host is selected from the group consisting of *Escherichia coli, Escherichia fergusonii, Anoxybacillus flavithermus* WK1, *Geobacillus thermodenitrificans* NG80-2, *Bacillus thermotolerans, Anoxybacillus flavithermus* AK1, *Methanocaldococcus jannaschii, Bacillus cereus,* and *Corynebacterium glutamicum.*

Those skilled in the art can prepare recombinantly engineered bacteria according to their actual needs by using a technical means well known in the art, see Molecular Cloning: A Laboratory Manual (3rd Edition) (Science Press). All of the vectors, plasmids, and hosts used in the experiment are the vectors, plasmids, and host series used for expression of conventional bacteria (such as *Escherichia coli),* such as vectors and plasmids within PET series, and host bacteria within BL21 series; the medium used is a medium for engineered bacteria of conventional bacteria (such as *Escherichia coli*)*,* such as LB medium; the culture method used is a culture method for engineered bacteria of conventional bacteria (such as *Escherichia coli*)*.*

In some embodiments, the bacteria expressing aspartase and L-aspartate-α-decarboxylase comprise bacteria expressing aspartase alone, and bacteria expressing L-aspartate-α-decarboxylase alone. In some embodiments, the bacteria are derived from wild-type bacteria or recombinantly engineered bacteria.

In some embodiments, the weight percentage of the bacteria expressing aspartase alone to the initial fumaric acid in the reactant is 0.5%-4% (w/w), for example, 0.6%, 0.7%, 0.8%, 0.9%, 1%, 1.1%, 1.2%, 1.3%, 1.4%, 1.5%, 1.6%, 1.7%, 1.8%, 1.9%, 2%, 2.5%, 3%, 3.5%, 4% or any value between any two values above, preferably 1%-2% (w/w); and the weight percentage of the bacteria expressing L-aspartate-α-decarboxylase alone to the initial fumaric acid in the reactant is 10%-30% (w/w), for example, 11%, 12%, 13%, 14%, 15%, 16%, 17%, 18%, 19%, 20%, 21%, 22%, 23%, 24%, 25%, 26%, 27%, 28%, 29%, 30% or any value between any two values above, preferably 15%-20% (w/w).

In some embodiments, when calculating the weight percentage of the bacteria to the initial fumaric acid, the weight of the bacteria is based on a wet weight. In some embodiments, when calculating the weight percentage of the bacteria to the initial fumaric acid, the weight of the bacteria is based on a dry weight. Those skilled in the art can make selections according to their actual needs. Those skilled in the art can also convert between a dry weight and a wet weight according to conventional means in the prior art, see for example, https://bionumbers.hms.harvard.edu/bionumber.aspx?id=109836.

In some embodiments, the activity of the aspartase and L-aspartate-α-decarboxylase produced by the recombinantly engineered bacteria used in the present invention is at least 10%, at least 15%, at least 20%, at least 25%, at least 30%, at least 35%, at least 40%, at least 45%, at least 50%, at least 55% higher, at least 60%, at least 65%, at least 70%, at least 75%, at least 80%, at least 85%, at least 90%, at least 95%, at least 100% and above higher than that of the aspartase and L-aspartate-α-decarboxylase produced by wild-type bacteria. Those skilled in the art can determine the activity of aspartase and L-aspartate-α-decarboxylase using a conventional technical means, for example, determination of the conversion rate of the substrate per unit time by using fumaric acid and aspartic acid as substrates, respectively.

Without being limited by theory, it is believed that the enzyme derived from the recombinantly engineered bacteria or the enzyme-expressing bacteria used in the present invention has unexpected advantages over wild-type bacteria. For example, compared with wild-type bacteria, the recombinantly engineered bacteria used in the present invention can greatly increase the reaction concentration of fumaric acid, for example, from 100 g/L to 200 g/L, while maintaining the high yield and high purity of the β-alanine product.

In some embodiments, the bacteria expressing aspartase and L-aspartate-α-decarboxylase comprise bacteria co-expressing aspartase and L-aspartate-α-decarboxylase. In some embodiments, the bacteria are derived from recombinantly engineered bacteria.

In some embodiments, the weight percentage of the bacteria co-expressing aspartase and L-aspartate-α-decarboxylase to the initial fumaric acid in the reactant is 10%-40% (w/w), such as 10%, 15%, 20%, 21%, 22%, 23%, 24%, 25%, 26%, 27%, 28%, 29%, 30%, 31%, 32%, 33%, 34%, 35%, 36%, 37%, 38%, 39%, 40% or any value between any two values above, preferably 20%-30% (w/w).

In the method for preparing β-alanine provided in the present application, the pH of the solution gradually increases with the progress of the catalytic reaction. The fumaric acid is added by the inventor of the present application during the reaction to control the pH value of the reaction. Without being limited by theory, it is believed that fumaric acid has obvious advantages over the conventional inorganic acid adjusters in the prior art in at least the following two aspects. In one aspect, fumaric acid can effectively reduce the pH value of the reaction solution into a suitable range. In the other aspect, the added fumaric acid can be used as a reaction substrate and is consumed by the catalytic reaction to generate β-alanine, which can avoid the introduction of additional impurities. On the contrary, if an inorganic acid adjuster is used to adjust the pH, it will form an ammonium salt of the inorganic acid with the aqueous ammonia in the reaction system, leading to the generation of impurities and the requirement of an additional impurity removal step.

In the method for preparing β-alanine provided in the present application, the intermediate aspartic acid is firstly produced by fumaric acid and aqueous ammonia under the catalysis of aspartase, and then the final product β-alanine is directly generated by aspartic acid under the catalysis of L-aspartate-α-decarboxylase without further purification or extraction, and this can achieve the purpose of directly generating β-alanine in one step without the extraction of the intermediate aspartic acid.

Another technical advantage of the present invention is that it can significantly reduce residual aqueous ammonia, fumaric acid and ammonium fumarate in the product. In the method for preparing β-alanine provided in the present application, the total moles of fumaric acid added are equal to the initial moles of aqueous ammonia in the reactant minus the initial moles of fumaric acid in the reactant, thereby ensuring the complete reaction of aqueous ammonia in the reactant and avoiding the existence of excessive fumaric acid in the product at the same time. In addition, aqueous ammonia and fumaric acid are converted into β-alanine upon a catalytic reaction, so that the content of uncatalyzed ammonium fumarate will also be significantly reduced.

In some embodiments of the present application, the initial molar ratio of fumaric acid to aqueous ammonia is designed to be 1:2, for example, it is assumed that the initial moles of fumaric acid are 1 mole, the initial moles of aqueous ammonia are 2 moles. In order to avoid pH increase during the reaction, the applicant's inventor cleverly controlled the pH value by adding fumaric acid during the reaction, and precisely controlled the amount of fumaric acid added during the reaction, so that the moles of fumaric acid added (1 mole) are equal to the initial moles of aqueous ammonia in the reactant (2 moles) minus the initial moles of fumaric acid in the reactant (1 mole), thereby ensuring the complete reaction of aqueous ammonia in the reactant and avoiding the existence of excessive fumaric acid in the product at the same time. The reaction process for preparing β-alanine from fumaric acid and aqueous ammonia can be summarized as follows:

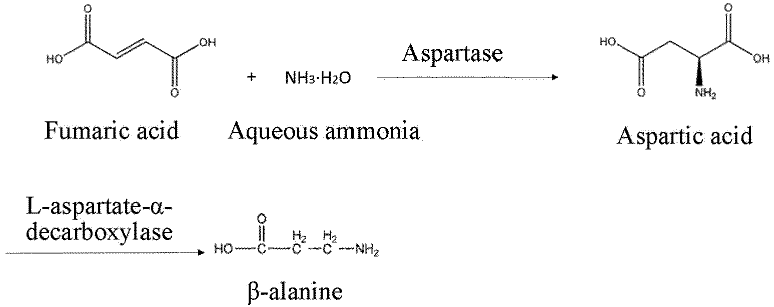

In some embodiments, the added fumaric acid is added in a fed-batch manner during the reaction. In some embodiments, the fed-batch speed of fumaric acid allows the pH value to be controlled at 6.8-7.2 during the reaction. In some embodiments, the concentration of fumaric acid is ranged from 50 to 400 g/L, and the fed-batch speed thereof allows the pH value to be controlled at 6.8-7.2 during the reaction, for example, the pH value of 6.8, 6.9, 7.0, 7.1, 7.2 or any value between any two values above. In some embodiments, the pH value of the reaction system can be detected in the fed-batch process, thereby adjusting the fed-batch speed of fumaric acid during the reaction. For example, in some embodiments, when the concentration is 100 g/L, the fed-batch speed depends on controlling the pH value at 6.8-7.1 during the reaction. In some embodiments, when the concentration is 200 g/L, the fed-batch speed depends on controlling the pH value at 6.9-7.2 during the reaction.

In some embodiments, the reaction temperature of the method for preparing β-alanine according to the present invention is controlled at 25°C-55°C, for example, 25°C, 30°C, 35°C, 36°C, 37°C, 38°C, 39°C, 40°C, 41°C, 42°C, 43°C, 44°C, 45°C, 46°C, 47°C, 48°C, 49°C, 50°C, 51°C, 52°C, 53°C, 54°C, 55°C or any value between any two values above, preferably, the reaction temperature is controlled at 35°C-42°C.

In some embodiments, after the catalytic reaction is completed, the method for preparing β-alanine according to the present invention further comprises removing residues in the catalytic composition.

In the present application, "catalytic reaction" refers to a reaction process when a β-alanine product is produced by reacting fumaric acid and aqueous ammonia in the presence of a catalyst. A variety of means can be used by those skilled in the art to determine whether the catalytic reaction is completed. In some embodiments, the reaction is monitored (for example, using HPLC) after the fed-batch process of fumaric acid is completed. When the content of fumaric acid is less than 0.5% (w/v), for example, 0.4%, 0.3%, 0.2%, 0.1% or even lower, and the molar conversion rate of aspartic acid is higher than 99%, for example, 99.1%, 99.2%, 99.3%, 99.4%, 99.5%, 99.6%, 99.7%, 99.8%, 99.9%, or even 100%, the reaction is considered to be completed. The molar conversion rate of aspartic acid can be calculated by those skilled in the art with a conventional means in the art, for example, the molar conversion rate of aspartic acid is determined by measuring the amount of each component in the reaction mixture with the HPLC method.

In some embodiments, the residues comprise large particles of impurities, such as cells, bacterial fragments, aggregates, flocs, and other impurities, as well as small molecular impurities, such as nucleic acids and nucleic acid fragments in the bacterial medium, proteins, medium components, and other impurities. The residues of the catalyst in the mixture can be removed by those skilled in the art with a conventional separation means according to their actual needs, for example, one or more of various means such as filtration, centrifugation, microfiltration and ultrafiltration.

In some embodiments, the filtration is achieved by using filter paper or filter cloth. The filter paper or filter cloth described in the present invention may be commercially available filter paper or filter cloth, such as those produced by GE Healthcare Life Sciences, Spectrum Laboratories Inc., or Asahi Kasei Corp. In some embodiments, the pore size of the filter paper or filter cloth is 10-150 µm, such as 10 µm, 20 µm, 30 µm, 40 µm, 50 µm, 60 µm, 70 µm, 80 µm, 90 µm, 10 0 µm, 110 µm, 120 µm, 130 µm, 140 µm, 150 µm or any value between any two values above. Those skilled in the art can select a suitable pore size of the filter paper or filter cloth to remove impurities according to the size of the impurities.

In some embodiments, the microfiltration is achieved by passing the reaction solution through a microfiltration membrane. The microfiltration membrane described in the present invention may be a commercially available microfiltration membrane, such as the microfiltration hollow fiber membrane series produced by GE Healthcare Life Sciences, Spectrum Laboratories Inc., or Asahi Kasei Corp. In some embodiments, the pore size of the microfiltration membrane is 0.1 µm to 0.6 µm, for example, 0.1 µm, 0.15 µm, 0.2 µm, 0.22 µm, 0.25 µm, 0.3 µm, 0.35 µm, 0.4 µm, 0.45 µm, 0.5 µm, 0.55 µm, 0.6 µm or any value between any two values above. Those skilled in the art can select a suitable pore size of the microfiltration membrane to remove the impurities according to the size of the impurities.

In some embodiments, the ultrafiltration is achieved by passing the reaction solution through an ultrafiltration membrane. The ultrafiltration membrane described in the present invention may be a commercially available ultrafiltration membrane, such as the ultrafiltration hollow fiber membrane series produced by GE Healthcare Life Sciences, Spectrum Laboratories Inc., or Asahi Kasei Corp. In some embodiments, the ultrafiltration membrane is a hollow fiber ultrafiltration membrane with a pore size of 5 kD-500 kD, for example, the hollow fiber ultrafiltration membrane with a pore size of 5 kD, 6 kD, 7 kD, 8 kD, 9 kD, 10 kD, 20 kD, 30 kD, 40 kD, 50 kD, 60 kD, 70 kD, 80 kD, 90 kD, 100 kD, 150 kD, 200 kD, 250 kD, 300 kD, 350 kD, 400 kD, 450 kD, 500 kD or any value between any two values above. Those skilled in the art can select a suitable pore size of the ultrafiltration membrane to remove impurities according to the size of the impurities.

In some embodiments, the method for preparing β-alanine according to the present invention further comprises concentrating the β-alanine product. In some embodiments, the concentration is achieved by reducing pressure, for example, the reaction solution after filtration, microfiltration or ultrafiltration is pumped into a concentration device to be concentrated under a reduced pressure to 1/3, 1/4, 1/5, 1/6, 1/7, 1/8, 1/9, 1/10 or any value between any two values above of the original volume.

In some embodiments, the method for preparing β-alanine according to the present invention further comprises crystallizing the β-alanine product. In some embodiments, the crystallization is achieved by lowering the temperature and adding organic solvents (such as methanol and ethanol), for example, adding 1 time, 2 times, 3 times, 4 times the same volume of the organic solvent dropwise to the concentrated reaction solution, and crystallizing under low temperature conditions (such as 10°C, 5°C or a lower temperature).

In the method for preparing β-alanine provided in the present application, a mother liquor is obtained after the crystallization. In the present application, "mother liquor" refers to the liquid remaining after the crystallization of β-alanine that is conducted after the catalytic reaction is completed. The content of inorganic salts in the mother liquor is less than 10 g/L, for example, less than 9 g/L, 8 g/L, 7 g/L, 6 g/L, 5 g/L, 4 g/L, 3 g/L, 2 g/L, 1 g/L and 0.5 g/L. In some embodiments, the mother liquor obtained in the crystallization process does not contain inorganic salts. The content of inorganic salts in the mother liquor can be determined by those skilled in the art with a conventional technical means, for example, the residual ammonium ions in the mother liquor is detected with an ammonia nitrogen detector (Shanghai INESA Scientific Instrument Co., Ltd.). Without being limited by theory, another technical advantage of the present invention is that the mother liquor remaining can be recycled after the reaction is completed since it basically does not contain impurities including inorganic salts, thereby avoiding the discharge of industrial waste water, and thus the environmental friendliness is increased. In some embodiments, β-alanine crystal is obtained after the crystallization, and the content of inorganic salts in the crystal is less than 20 mg/g, for example, less than 19 mg/g, 18 mg/g, 17 mg/g, 16 mg/g, 15 mg/g, 14 mg/g, 13 mg/g, 12 mg/g, 11 mg/g, 10 mg/g, 9 mg/g, 8 mg/g, 7 mg/g, 6 mg/g, 5 mg/g, 4 mg/g, 3 mg/g, 2 mg/g and 1 mg/g.

Compared with the prior art, the method for preparing β-alanine according to the present invention has the following advantages:
1. low-priced fumaric acid is used as the initial substrate to directly generate β-alanine in one step without intermediate extraction of aspartic acid, which simplifies the production process, reduces production costs, and is environmentally friendly;
2. the thermostable enzymes is efficiently expressed in engineered bacteria, wherein the enzyme activity is high, the enzyme stability is good, the reaction substrate concentration is high, the conversion efficiency is high, and the molar conversion rates of fumaric acid and aspartic acid are both greater than 99%;
3. the pH value is controlled by the fed-batch of fumaric acid, other inorganic acids (such as phosphoric acid, sulfuric acid, hydrochloric acid or nitric acid) are not introduced in the entire reaction process, and the moles of fumaric acid, the moles of aqueous ammonia, and the moles of fumaric acid added via the fed-batch manner in the reactant during the reaction are precisely controlled, so that no by-products and inorganic salts are generated during the reaction, the product extraction process is simple, and the product purity is high; and
4. the mother liquor obtained after the crystallization of β-alanine contains almost no inorganic salts and other impurities, and the mother liquor can be recycled in theory, thereby reducing the production cost.

In another aspect, the present invention provides a method for preparing β-alanine salt, comprising the following steps of:
(a) preparing β-alanine by the method according to the present invention; and
(b) reacting the β-alanine obtained in step (a) with an alkaline solution.

Specifically, in some embodiments, the method for preparing β-alanine salt according to the present invention comprises the following steps of:
(a) preparing a β-alanine product from a reactant containing fumaric acid and aqueous ammonia in the presence of a catalyst, wherein the catalyst includes a catalytic composition containing aspartase and L-aspartate-α-decarboxylase, and adding fumaric acid during the reaction, wherein the total moles of the fumaric acid added is equal to the initial moles of the aqueous ammonia in the reactant minus the initial moles of the fumaric acid in the reactant; and
(b) reacting the β-alanine obtained in step (a) with an alkaline solution.

In some embodiments, the β-alanine is dissolved in the solvent before reacting the β-alanine obtained in step (a) with the alkaline solution. In some embodiments, the β-alanine is dissolved in water before reacting the β-alanine obtained in step (a) with an alkaline solution.

In some embodiments, the β-alanine salt according to the present invention is an alkali metal salt of β-alanine, for example, alkali metal salt containing cations of any one of lithium (Li), sodium (Na), potassium (K), rubidium (Rb), and cesium (Cs). In some embodiments, the β-alanine salt is sodium β-alanine or potassium β-alanine. In some embodiments, the β-alanine salt according to the present invention is an alkaline earth metal salt of β-alanine, for example, alkaline earth metal containing cations of any one of beryllium (Be), magnesium (Mg), calcium (Ca), strontium (Sr), barium (Ba), and radium (Ra). In some embodiments, the β-alanine salt according to the present invention is calcium β-alanine.

In the present application, "alkaline solution" refers to a solution with a pH>7 at room temperature, or with higher hydroxide ion concentration than hydrogen ion concentration. In some embodiments, the property of the alkaline solution selected should allow reacting with β-alanine under suitable conditions. In some embodiments, the alkaline solution is one containing alkali metal or alkaline earth metal cations. In some embodiments, the alkaline solution comprises NaOH, KOH, Ca(OH)₂, Mg(OH)₂, Al(OH)₃, or a combination thereof.

Depending on the target β-alanine salt, those skilled in the art can select a suitable alkaline solution for the reaction. In some embodiments, the alkaline solution can also be used immediately. For example, during preparing the calcium salt of β-alanine, calcium oxide is added to the aqueous solution of β-alanine, and then reacts with water to produce Ca(OH)₂, which reacts with β-alanine to produce calcium salt of β-alanine (the specific reaction process is shown below). In this preparation method, the feeding ratio (weight ratio) of β-alanine, calcium oxide and water is between 3:1:5 and 3:1:17, such as 3:1:6, 3:1:7, 3:1:8, 3:1:9, 3:1:10, 3:1:11, 3:1:12, 3:1:13, 3:1:14, 3:1:15 and 3:1:16. In some embodiments, the feeding ratio (weight ratio) of β-alanine, calcium oxide and water is between 3:1:6 and 3:2:6, such as 3:1.1:6, 3:1.2: 6, 3:1.3:6, 3:1.4:6, 3:1.5:6, 3:1.6:6, 3:1.7:6, 3:1.8:6, 3:1.9:6 and 3:2:6.

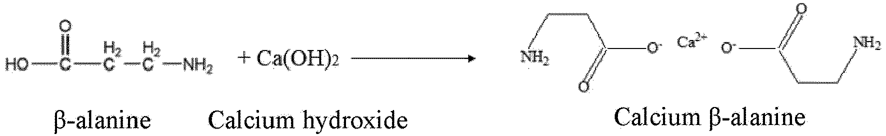

In some embodiments, step (b) in the method for preparing β-alanine salt according to the present invention is carried out under suitable conditions. For example, in some embodiments, the reaction temperature of step (b) is controlled at 40°C-100°C, for example, 40°C, 50°C, 60°C, 70°C, 80°C, 85°C, 90°C, 95°C, 100°C or any value between any two values above, preferably the reaction temperature is controlled at 80°C-100°C, for example, 85°C, 86°C, 87°C, 88°C, 89° C, 90°C, 91°C, 92°C, 93°C, 94°C, 95°C, 96°C, 97°C, 98°C, 99°C and 100°C.

In some embodiments, in order to increase the yield of β-alanine salt, a temperature control method of increasing temperature-decreasing temperature-increasing temperature again is adopted in the reaction process of step (b), so as to complete the reaction. For example, the reaction solution is first heated to 80°C-100°C (for example, 85°C, 86°C, 87°C, 88°C, 89°C, 90°C, 91°C, 92°C, 93°C, 94°C, 95°C, 96°C, 97°C, 98°C, 99°C, and 100°C), then cooled to 50°C-70°C (for example, 55°C, 56°C, 57° C, 58°C, 59°C, 60°C, 61°C, 62°C, 63°C, 64°C, 65°C, 66°C, 67°C, 68°C, 69°C, and 70°C), kept at the same temperature for a certain period of time (for example, from 30 minutes-1 hour), and filtered while the reaction solution is warm, and then the filtrate is heated to 80°C-100°C (for example, 85°C, 86°C, 87°C, 88°C, 89°C, 90°C, 91°C, 92°C, 93°C, 94°C, 95°C, 96°C, 97°C, 98°C, 99°C, and 100°C), kept at the same temperature for a certain period of time (for example, 30 minutes-1 hour), and then filtered again until the solid precipitates out.

In some embodiments, the method for preparing the β-alanine salt according to the present invention further comprises concentrating the β-alanine salt product. In some embodiments, the concentration is achieved by reducing pressure, for example, the reaction solution after filtration, microfiltration or ultrafiltration is pumped into a concentration device to be concentrated under a reduced pressure to 1/3, 1/4, 1/5, 1/6, 1/7, 1/8, 1/9, 1/10 or any value between any two values above of the original volume.

In some embodiments, the method for preparing β-alanine salt according to the present invention further comprises crystallizing the β-alanine salt product. In some embodiments, the crystallization is achieved by lowering the temperature and adding organic solvents (for example, methanol, ethanol and isopropanol) or water, for example, adding 1-10 times (for example, 1 time, 2 times, 3 times, 4 times, 5 times, 6 times, 7 times, 8 times, 9 times and 10 times) mass of the organic solvent or water dropwise to the concentrated reaction solution and crystallizing under low temperature conditions of 0°C-10°C (for example, 10°C, 9°C, 8°C, 7°C, 6°C, 5°C, 4°C, 3°C, 2°C, 1°C, and 0°C) or a lower temperature.

In some embodiments, the method for preparing β-alanine salt according to the present invention comprises: charging the β-alanine obtained by the method of the present invention and a solvent (for example, water) into a container, then adding an alkaline solution or calcium oxide then heating the container to 80°C-100°C and refluxing for 0.5-5 hours, then cooling to 50°C-70°C, keeping at the same temperature for 0.5-5 hours, filtering, and then heating the filtrate to 80°C-100°C, keeping at the same temperature for 0.5-5 hours, filtering and crystallizing.

Compared with the prior art, the method for preparing β-alanine salt according to the present invention has the following advantages:
(1) it can reduce costs to a greater extent and is suitable for industrial production; and
(2) in the traditional preparation process of calcium β-alanine, the usual method involves dissolving β-alanine obtained by using inorganic acid adjuster and calcium oxide in methanol for reaction, rather than choosing water as the solvent, since water will dissolve salty impurities introduced in the traditional preparation process of β-alanine. Therefore, the method for preparing β-alanine salt according to the present invention not only avoids the introduction of additional impurities, thereby affecting the synthesis of pantothenate, but also avoids the use of organic solvents (for example, methanol), thereby reducing production costs and achieving greenness and environmental friendliness.

In another aspect, the present invention provides a method for preparing pantothenate, comprising the following steps of:
(a) preparing β-alanine by the method according to the present invention;
(b) reacting the β-alanine obtained in step (a) with an alkaline solution to prepare β-alanine salt; and
(c) reacting pantolactone or pantoic acid with the β-alanine salt obtained in step (b).

Specifically, in some embodiments, the method for preparing pantothenate according to the present invention comprises the following steps of:
(a) preparing a β-alanine product from a reactant containing fumaric acid and aqueous ammonia in the presence of a catalyst, wherein the catalyst includes a catalytic composition containing aspartase and L-aspartate-α-decarboxylase, and adding fumaric acid during the reaction, wherein the total moles of the fumaric acid added is equal to the initial moles of the aqueous ammonia in the reactant minus the initial moles of the fumaric acid in the reactant;
(b) reacting the β-alanine obtained in step (a) with an alkaline solution to prepare β-alanine salt; and
(c) reacting pantolactone or pantoic acid with the β-alanine salt obtained in step (b).

Pantolactone is an organic compound of γ-butyrolactones and is an intermediate of pantothenic acid. In some embodiments, the pantolactone used in the present invention is DL-pantolactone (i.e., pantolactone without optical activity). In some embodiments, the pantolactone used in the present invention is D-pantolactone (the specific reaction process is shown below).

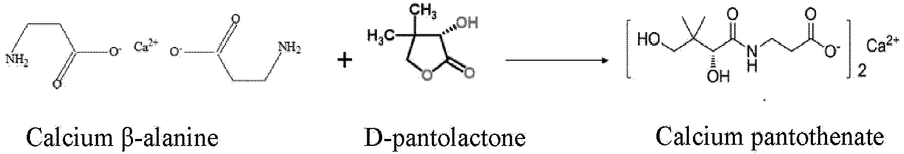

Pantoic acid is a ring-opened form of pantolactone. In some embodiments, the pantoic acid used in the present invention is DL-pantoic acid (i.e., pantoic acid without optical activity). In some embodiments, the pantoic acid used in the present invention is D-pantoic acid.

In some embodiments, the pantolactone or pantoic acid is dissolved in a solvent (for example, water, methanol, ethanol, isopropanol, n-propanol, butanol, pentanol, ether, benzene and chloroform) before reaction with the β-alanine salt obtained by the method of the present invention. In some embodiments, the pantolactone or pantoic acid is dissolved in methanol or ethanol before reaction with the β-alanine salt obtained by the method of the present invention. In some embodiments, the pantolactone or pantoic acid is dissolved in methanol before reaction with the β-alanine salt obtained by the method of the present invention.

In some embodiments, the feeding ratio (molar ratio) of pantolactone (for example, D-pantolactone) to β-alanine salt is between 2:1 and 2:1.5, such as 2:1.05, 2:1.1, 2:1.15, 2:1.2, 2:1.25, 2:1.3, 2:1.35, 2:1.4, 2:1.45 and 2:1.5. In some embodiments, the feeding ratio (mass ratio) of the solvent (for example, methanol) and pantolactone (for example, D-pantolactone) is between 2:1 and 5:1, such as 2:1, 2.5:1, 3:1, 3.5:1, 3.6:1, 3.7:1, 3.8:1, 3.9:1, 4:1, 4.1:1, 4.2:1, 4.3:1, 4.4:1, 4.5:1, 4.6:1, 4.7:1, 4.8:1, 4.9:1 and 5:1.

In some embodiments, the method for preparing pantothenate according to the present invention further comprises filtering and/or drying the β-alanine salt obtained in step (b) before step (c). One of the advantages of this approach is that it can remove the water produced during the preparation of β-alanine salt, thereby avoiding the influence of water on the formation of pantothenate (for example, calcium pantothenate, sodium pantothenate, and potassium pantothenate) in step (c) and greatly improving the quality of pantothenate (for example, calcium pantothenate, sodium pantothenate, and potassium pantothenate).

In some embodiments, step (c) in the method for preparing pantothenate according to the present invention is carried out under suitable reaction conditions. In some embodiments, the reaction temperature of step (c) is controlled at 40°C-100°C, for example, 40°C, 50°C, 60°C, 70°C, 80°C, 85°C, 90°C, 95°C, 100°C or any value between any two values above, preferably, the reaction temperature is controlled at 40°C-60°C.

In some embodiments, the method for preparing pantothenate according to the present invention further comprises concentrating the pantothenate product obtained in step (c). In some embodiments, the concentration is achieved by reducing pressure, for example, the reaction solution after filtration, microfiltration or ultrafiltration is pumped into a concentration device to be concentrated under a reduced pressure to 1/3, 1/4, 1/5, 1/6, 1/7, 1/8, 1/9, 1/10 or any value between any two values above of the original volume.

In some embodiments, the method for preparing pantothenate according to the present invention further comprises crystallizing the pantothenate product obtained in step (c). In some embodiments, the crystallization is achieved by lowering the temperature and adding organic solvents (for example, methanol, ethanol and isopropanol) or water, for example, adding a small amount of crystal water to the concentrated reaction solution and crystallizing under low temperature conditions (for example, 0°C, -5°C, -6°C, -7°C, -8°C, - 9°C, -10°C, -11°C, -12°C, -13°C, -14°C, -15°C or a lower temperature). Without being bound by any theory, it is believed that the lower the temperature, the better the crystallization of the pantothenate product, and the higher the yield of the pantothenate.

In some embodiments, the method for preparing pantothenate according to the present invention comprises charging pantolactone (for example, D-pantolactone) and a solvent (for example, methanol) into a container, then adding the β-alanine salt obtained by the method of the present invention (for example, calcium β-alanine, sodium β-alanine, and potassium β-alanine) before heating to 40°C-60°C, reacting for 1-20 hours, filtering and crystallizing.

In another aspect, the present invention provides a calcium pantothenate, wherein content of chloride ion (by weight) is not higher than 190 ppm, and/or content of a sodium ion (by weight) is not higher than 2200 ppm. In some embodiments, the content of chloride ions (by weight) in calcium pantothenate provided in the present invention is no higher than 180 ppm, no higher than 170 ppm, no higher than 160 ppm, no higher than 150 ppm, no higher than 140 ppm, no higher than 130 ppm, no higher than 120 ppm, no higher than 110 ppm, no higher than 100 ppm, no higher than 90 ppm, no higher than 80 ppm, no higher than 70 ppm, no higher than 60 ppm, no higher than 50 ppm, no higher than 40 ppm, no higher than 30 ppm, no more than 20 ppm, and no more than 10 ppm. For example, in some embodiments, the content of chloride ions (by weight) in calcium pantothenate provided in the present invention is 1 ppm-190 ppm, 1 ppm-180 ppm, 1 ppm-170 ppm, 1 ppm-160 ppm, 1 ppm-150 ppm, 1 ppm-140 ppm, 1 ppm-130 ppm, 1 ppm-120 ppm, 1 ppm-110 ppm, 1 ppm-100 ppm, 1 ppm-90 ppm, 1 ppm-80 ppm, 1 ppm-70 ppm, 1 ppm-60 ppm, 1 ppm-50 ppm, 1 ppm-40 ppm, 1 ppm-30 ppm, 1 ppm-20 ppm, 1 ppm-10 ppm, or any value between any two numerical ranges above.

In some embodiments, the content of sodium ions (by weight) in calcium pantothenate provided in the present invention is no higher than 2150 ppm, no higher than 2100 ppm, no higher than 2050 ppm, no higher than 2000 ppm, no higher than 1950 ppm, not higher 1900 ppm, no higher than 1850 ppm, and no higher than 1800 ppm. For example, in some embodiments, the content of sodium ions (by weight) in calcium pantothenate provided in the present invention is 1800 ppm-2200 ppm, 1800 ppm-2150 ppm, 1800 ppm-2100 ppm, 1800 ppm-2050 ppm, 1800 ppm-2000 ppm, 1800 ppm-1950 ppm, 1800 ppm-1900 ppm, 1800 ppm-1850 ppm, or any value between any two numerical ranges above.

In some embodiments, calcium pantothenate with the content of chloride ions (by weight) no higher than 190 ppm and/or the content of sodium ions (by weight) no higher than 2200 ppm is obtained by the method according to the present invention. In some embodiments, the method for preparing calcium pantothenate comprises the following steps of:
(a) preparing β-alanine by the method according to the present invention;
(b) reacting the β-alanine obtained in step (a) with an alkaline solution containing calcium salt to prepare calcium β-alanine; and
(c) reacting pantolactone or pantoic acid with the calcium β-alanine obtained obtainedin step (b).

Specifically, in some embodiments, the method for preparing calcium pantothenate according to the present invention comprises the following steps of:
(a) preparing a β-alanine product from a reactant containing fumaric acid and aqueous ammonia in the presence of a catalyst, wherein the catalyst includes a catalytic composition containing aspartase and L-aspartate-α-decarboxylase, and adding fumaric acid during the reaction, wherein the total moles of the fumaric acid added is equal to the initial moles of the aqueous ammonia in the reactant minus the initial moles of the fumaric acid in the reactant; and;
(b) reacting the β-alanine obtained in step (a) with an alkaline solution containing calcium salt (for example, calcium hydroxide) to prepare calcium β-alanine; and
(c) reacting pantolactone or pantoic acid with the calcium β-alanine obtained in step (b).

The present invention will be further described below in conjunction with specific examples, but the protection scope of the present invention is not limited thereto.

### Examples

### Example 1: Fermentation culture of engineered Escherichia coli

Fermentation culture of engineered *Escherichia coli:* a formulation for inorganic salts in fermentation medium having NaCl 1 g/L; MgSO₄-7H₂O 1 g/L; FeSO₄-7H₂O 0.02 g/L; ZnSO₄-7H₂O 0.03 g/L; CuSO₄-5H₂O 0.005 g/L; (NH₄)₂SO₄ 4 g/L; and KH₂PO₄ 4 g/L, was sterilized at 121°C for 15 minutes, cooled to 37°C after sterilization, inoculated with an inoculum size of 2%, and fermented at 37°C with a dissolved oxygen of 30%-50% and aeration of 1 VVM. Aqueous ammonia was added in a fed-batch manner to adjust pH to 7.5 and provide a nitrogen source, glucose was added in a fed-batch manner to provide a carbon source, residual sugar was controlled at 0.1-0.5 g/L. Upon culturing to OD₆₀₀ = 30, the temperature was lowered to 30°C and then IPTG was added for induction. Fermentation was terminated after inducing culture for 30 hours. Upon reaching OD₆₀₀ of about 110, centrifugation was performed to provide the bacteria with wet weight of about 120 g/L.

### Example 2: Synthesis of β-alanine

### Example 2.1

Engineered *Escherichia coli* strain containing aspartase (hereinafter referred to as "enzyme 1") derived from *Anoxybacillus flavithermus* WK1 and engineered *Escherichia coli*strain containing L-aspartate-α-decarboxylase (hereinafter referred to as "enzyme 2") derived from *Bacillus thermotolerans* were prepared with a reference to Molecular Cloning: A Laboratory Manual (3rd Edition) (China Science Publishing & Media) and Li Y. et. al. Appl. Microbiol. Biotechnol. 2017, 101, 6015-6021, fermented with a conventional LB medium or the method of Example 1 and then centrifuged (rotation speed: 5000 rpm, centrifugation time: 5 min) to obtain a wet engineered *Escherichia coli* for later use.

3 L ammonium fumarate solution (100 g fumaric acid/L) with the initial moles of fumaric acid of 2.586 moles and the initial moles of aqueous ammonia of 5.172 moles was obtained, and 3 g of wet engineered *Escherichia coli* containing enzyme 1 derived from *Anoxybacillus flavithermus* WK1 (the weight ratio of the wet bacteria to the initial fumaric acid was 1%) and 60 g of wet engineered *Escherichia coli* containing enzyme 2 derived from *Bacillus thermotolerans* (the weight ratio of the wet bacteria to the initial fumaric acid was 20%) were added to start the reaction. 2.586 moles of fumaric acid (100 g fumaric acid/L) (plus the initial moles of fumaric acid in the reactants equaled to 5.172 moles of fumaric acid in total) was added in a fed-batch manner to control the pH value of the reaction at 7.0, the reaction temperature was 37°C, and the reaction was monitored by HPLC at the end of the addition of the fumaric acid in a fed-batch manner. When the reaction time was 18 h, the fumaric acid content is less than 0.5% (w/v), and the molar conversion rate of aspartic acid was more than 99%, the reaction was terminated.

The reaction solution was passed through a 0.4 µm microfiltration membrane to remove large particles of impurities, such as cells, bacterial fragments, aggregates, flocs and other impurities, and passed through a 10 KD ultrafiltration membrane to remove small molecular impurities, such as nucleic acids and nucleic acid fragments in the bacterial medium, proteins, medium components, and other impurities, and concentrated under a reduced pressure to 1/5 of the volume of the original reaction solution. 3 times the volume of methanol was added dropwise to crystallize at a low temperature of 10°C, and the mixture was suction filtered and dried to obtain a 390.5 g white solid with the β-alanine content of 98.7% and the yield of 83.7%. The methanol in the mother liquor was recovered and then recycled.

### Example 2.2

Engineered *Escherichia coli* strain containing enzyme 1 derived from *Geobacillus thermodenitrificans* NG80-2 and engineered *Escherichia coli* strain containing enzyme 2 derived from *Anoxybacillus flavithermus* AK1 were obtained with a reference to the same references in Example 2.1, fermented with a conventional LB medium or the method of Example 1 and then centrifuged (rotation speed: 5000 rpm, centrifugation time: 5 min) to obtain wet engineered *Escherichia coli.* 10 ml/1 g cell of phosphate buffer at pH 7.0 was added, the mixture was stirred evenly, and the bacteria were disrupted by high-pressure homogenization to obtain a disrupted liquid of engineered *Escherichia coli* for later use.

3 L ammonium fumarate solution (150 g fumaric acid/L) with the initial moles of fumaric acid of 3.879 moles and the initial moles of aqueous ammonia of 7.758 moles was obtained, and 9 g of disrupted liquid of engineered *Escherichia coli* containing enzyme 1 derived from *Geobacillus thermodenitrificans* NG80-2 (the weight ratio of the disrupted liquid to the initial fumaric acid was 2%) and 135 g of disrupted liquid of engineered *Escherichia coli* containing enzyme 2 derived from *Anoxybacillus flavithermus* AK1 (the weight ratio of the disrupted liquid to the initial fumaric acid was 30%) were added to start the reaction. 3.879 moles of fumaric acid (150 g fumaric acid/L) (plus the initial moles of fumaric acid in the reactants equaled to 7.758 moles of fumaric acid in total) was added in a fed-batch manner to control the pH value of the reaction at 7.0, the reaction temperature was 37°C, and the reaction was monitored by HPLC at the end of the addition of the fumaric acid in a fed-batch manner. When the reaction time was 24 h, the fumaric acid content is less than 0.5% (w/v), and the molar conversion rate of aspartic acid was more than 99%, the reaction was terminated.

The reaction solution was passed through a 0.4 µm microfiltration membrane to remove large particles of impurities, such as cells, bacterial fragments, aggregates, flocs and other impurities, and passed through a 6 KD ultrafiltration membrane to remove small molecular impurities, such as nucleic acids and nucleic acid fragments in the bacterial medium, proteins, medium components, and other impurities, and concentrated under a reduced pressure to 1/4 of the volume of the original reaction solution. 3 times the volume of methanol was added dropwise to crystallize at a low temperature of 5°C, and the mixture was suction filtered and dried to obtain a 622.5 g white solid with the β-alanine content of 99.1% and the yield of 89.3%. The methanol in the mother liquor was recovered and then recycled.

### Example 2.3

Engineered *Escherichia coli* strain containing enzyme 1 derived from *Anoxybacillus flavithermus* WK1 and engineered *Escherichia coli* strain containing enzyme 2 derived from *Methanocaldococcus jannaschii* were obtained with a reference to the same references in Example 2.1, fermented with a conventional LB medium or the method of Example 1 and then centrifuged (rotation speed: 5000 rpm, centrifugation time: 5 min) to obtain a wet engineered *Escherichia coli* for later use.

3 L ammonium fumarate solution (75 g fumaric acid/L) with the initial moles of fumaric acid of 1.939 moles and the initial moles of aqueous ammonia of 3.878 moles was obtained, 20 mM pyridoxal phosphate (PLP) was added, and 2.25 g of wet engineered *Escherichia coli* containing enzyme 1 derived from *Anoxybacillus flavithermus* WK1 (the weight ratio of the wet bacteria to the initial fumaric acid was 1%) and 33.75 g of wet engineered *Escherichia coli* containing enzyme 2 derived from *Methanocaldococcus jannaschii* (the weight ratio of the wet bacteria to the initial fumaric acid was 15%) were added to start the reaction. 1.939 moles of fumaric acid (75 g fumaric acid/L) (plus the initial moles of fumaric acid in the reactants equaled to 3.878 moles of fumaric acid in total) was added in a fed-batch manner to control the pH value of the reaction at 7.0, the reaction temperature was 37°C, and the reaction was monitored by HPLC at the end of the addition of the fumaric acid in a fed-batch manner. When the reaction time was 24 h, the fumaric acid content is less than 0.5% (w/v), and the molar conversion rate of aspartic acid was more than 99%, the reaction was terminated.

The reaction solution was passed through a 0.4 µm microfiltration membrane to remove large particles of impurities, such as cells, bacterial fragments, aggregates, flocs and other impurities, and passed through a 10 KD ultrafiltration membrane to remove small molecular impurities, such as nucleic acids and nucleic acid fragments in the bacterial medium, proteins, medium components, and other impurities, and concentrated under a reduced pressure to 1/7 of the volume of the original reaction solution. The temperature was decreased slowly to 4°C to crystallize at a low temperature and the mixture was suction filtered and dried to obtain a 227.6 g white solid with the β-alanine content of 99.4% and the yield of 65.5%. The methanol in the mother liquor was recovered and then recycled.

### Example 2.4

Engineered *Escherichia coli* strain co-expressing enzyme 1 derived from *Anoxybacillus flavithermus* WK1 and enzyme 2 derived from *Bacillus thermotolerans* was obtained with a reference to the same references in Example 2.1, fermented with a conventional LB medium or the method of Example 1 and then centrifuged (rotation speed: 5000 rpm, centrifugation time: 5 min) to obtain a wet engineered *Escherichia coli* for later use.

3 L ammonium fumarate solution (100 g fumaric acid/L) with the initial moles of fumaric acid of 2.586 moles and the initial moles of aqueous ammonia of 5.172 moles was obtained, and 90 g of wet engineered *Escherichia coli* co-expressing enzyme 1 derived from *Anoxybacillus flavithermus* WK1 and enzyme 2 derived from *Bacillus thermotolerans* (the weight ratio of the wet bacteria to the initial fumaric acid was 30%) were added to start the reaction. 2.586 moles of fumaric acid (100 g fumaric acid/L) (plus the initial moles of fumaric acid in the reactants equaled to 5.172 moles of fumaric acid in total) was added in a fed-batch manner to control the pH value of the reaction at 7.0, the reaction temperature was 37°C, and the reaction was monitored by HPLC at the end of the addition of the fumaric acid in a fed-batch manner. When the reaction time was 27 h, the fumaric acid content is less than 0.5% (w/v), and the molar conversion rate of aspartic acid was more than 99%, the reaction was terminated.

The reaction solution was passed through a 0.4 µm microfiltration membrane to remove large particles of impurities, such as cells, bacterial fragments, aggregates, flocs and other impurities, and passed through a 10 KD ultrafiltration membrane to remove small molecular impurities, such as nucleic acids and nucleic acid fragments in the bacterial medium, proteins, medium components, and other impurities, and concentrated under a reduced pressure to 1/6 of the volume of the original reaction solution. The temperature was decreased slowly to 5°C to crystallize at a low temperature and the mixture was suction filtered and dried to obtain a 338.5 g white solid with the β-alanine content of 99.3% and the yield of 73%. The methanol in the mother liquor was recovered and then recycled.

### Example 2.5

*Escherichia coli* cells co-expressing enzyme 1 derived from *Anoxybacillus flavithermus* WK1 and enzyme 2 derived from *Bacillus thermotolerans* were obtained with a reference to the same references in Example 2.1 and fermented with a conventional LB medium or the method of Example 1. After that, a mixed solution of 8% polyvinyl alcohol and 2.5% sodium alginate was obtained, 10% *Escherichia coli* cells were added, mixed well, and then added dropwise with a peristaltic pump into a crosslinking agent containing 2% calcium chloride and 3% boric acid from a height of 10 cm. The mixture was solidified for 8 hours, filtered, and repeatedly rinsed with distilled water for 3-4 times, and the cells were immobilized for later use.

3 L ammonium fumarate solution (75 g fumaric acid/L) with the initial moles of fumaric acid of 1.939 moles and the initial moles of aqueous ammonia of 3.878 moles was obtained, and 67.5 g of immobilized *Escherichia coli* cells co-expressing enzyme 1 derived from *Anoxybacillus flavithermus* WK1 and enzyme 2 derived from *Bacillus thermotolerans* (the weight ratio of the immobilized cells to the initial fumaric acid was 30%) were added to start the reaction. 1.939 moles of fumaric acid (75 g fumaric acid/L) (plus the initial moles of fumaric acid in the reactants equaled to 3.878 moles of fumaric acid in total) was added in a fed-batch manner to control the pH value of the reaction at 7.0, the reaction temperature was 37°C, and the reaction was monitored by HPLC at the end of the addition of the fumaric acid in a fed-batch manner. When the reaction time was 20 h, the fumaric acid content is less than 0.5% (w/v), and the molar conversion rate of aspartic acid was more than 99%, the reaction was terminated.

The reaction solution was passed through filter cloth to remove large particles of impurities, such as cells, bacterial fragments, aggregates, flocs and other impurities, and passed through a 10 KD ultrafiltration membrane to remove small molecular impurities, such as nucleic acids and nucleic acid fragments in the bacterial medium, proteins, medium components, and other impurities, and concentrated under a reduced pressure to 1/7 of the volume of the original reaction solution. 3 times the volume of methanol was added dropwise to crystallize at a low temperature of 10°C, and the mixture was suction filtered and dried to obtain a 296.7 g white solid with the β-alanine content of 98.9% and the yield of 85%. The methanol in the mother liquor was recovered and then recycled.

### Example 3: Comparison of salt-free process and salt process

The preparation method according to the present invention (hereinafter referred to as "salt-free process") and the preparation method with appropriate inorganic acids (hereinafter referred to as "salt process") were respectively used by the applicant to prepare β-alanine, and the contents of β-alanine, ammonia nitrogen and ammonia nitrogen per gram of β-alanine obtained by the two processes were compared.

Salt-free process: *Escherichia coli* whole cells containing enzyme 1 derived from *Anoxybacillus flavithermus* WK1 and *Escherichia coli* whole cells containing enzyme 2 derived from *Bacillus thermotolerans* were obtained with a reference to the same references in Example 2.1 and fermented with a conventional LB medium or the method of Example 1 for later use.

3 L ammonium fumarate solution (100 g fumaric acid/L) with the initial moles of fumaric acid of 2.586 moles and the initial moles of aqueous ammonia of 5.172 moles was obtained, and 3 g of *Escherichia coli* whole cells containing enzyme 1 derived from *Anoxybacillus flavithermus* WK1 (the weight ratio of the whole cells to the initial fumaric acid was 1%) and 60 g of *Escherichia coli* whole cells containing enzyme 2 derived from *Bacillus thermotolerans* (the weight ratio of the whole cells to the initial fumaric acid was 20%) were added to start the reaction. 2.586 moles of fumaric acid (100 g fumaric acid/L) (plus the initial moles of fumaric acid in the reactants equaled to 5.172 moles of fumaric acid in total) was added in a fed-batch manner to control the pH value of the reaction at 7.0, the reaction temperature was 37°C, and the reaction was monitored by HPLC at the end of the addition of the fumaric acid in a fed-batch manner. When the reaction time was 18 h, the fumaric acid content is less than 0.5% (w/v), and the molar conversion rate of aspartic acid was more than 99%, the reaction was terminated. An ammonia nitrogen detector (Shanghai INESA Scientific Instrument Co., Ltd.) was used to detect the ammonium ion concentration in the reaction solution to be 0.82 g/L.

### Control experiment

Salt process: *Escherichia coli* whole cells containing enzyme 1 derived from *Anoxybacillus flavithermus* WK1 and *Escherichia coli* whole cells containing enzyme 2 derived from *Bacillus thermotolerans* were obtained with a reference to the same references in Example 2.1 and fermented with a conventional LB medium or the method of Example 1 for later use.

3 L ammonium fumarate solution (200 g fumaric acid/L) with the initial moles of fumaric acid of 5.172 moles and the initial moles of aqueous ammonia of 10.344 moles was obtained, and 6 g of *Escherichia coli* whole cells containing enzyme 1 derived from *Anoxybacillus flavithermus* WK1 (the weight ratio of the whole cells to the initial fumaric acid was 1%) and 120 g of *Escherichia coli* whole cells containing enzyme 2 derived from *Bacillus thermotolerans* (the weight ratio of the whole cells to the initial fumaric acid was 20%) were added to start the reaction. H₂SO₄ was added in a fed-batch manner to control the pH value of the reaction at 7.0, the reaction temperature was 37°C, and the reaction was monitored by HPLC. When the reaction time was 18 h, the fumaric acid content is less than 0.5% (w/v), and the molar conversion rate of aspartic acid was more than 99%, the reaction was terminated. An ammonia nitrogen detector (Shanghai INESA Scientific Instrument Co., Ltd.) was used to detect the ammonium ion concentration in the reaction solution to be 23.5 g/L.

Both the salt-free process and the salt process used the same after-treatment process. Large particles of impurities, such as cells, bacterial fragments, aggregates, flocs and other impurities, were removed through a 0.4 µm microfiltration membrane, and small molecular impurities, such as nucleic acid and nucleic acid fragments in the bacterial medium, proteins, medium components and other impurities, were removed through a 10 KD ultrafiltration membrane. The residue was concentrated under a reduced pressure to 1/5 of the volume of the original reaction solution, crystallized at a low temperature of 10°C, suction filtered and dried. The results were as follows:
(1) in the salt-free process, 207 g of β-alanine was obtained with a content of 98.6%, and the ammonia nitrogen content per gram of β-alanine was 13 mg/g; and the ammonia nitrogen content in the recovered liquid was 2.3 g/L; and
(2) in the salt process, 290 g of β-alanine was obtained with a content of 55.6%, and the ammonia nitrogen content per gram of β-alanine was 910 mg/g; and the ammonia nitrogen content in the recovered liquid was 51.7 g/L.

### Example 4: Recycling of salt-free process

The preparation method according to the present invention was used by the applicant to recycle the salt-free process.

3 batches of reaction solutions were obtained by the method of the salt-free process in Example 2.5, and were numbered A, B, and C, respectively.
(1) The reaction solution A was concentrated under a reduced pressure to 1/5 of the volume of the original reaction solution, crystallized at a low temperature of 10°C, suction filtered and dried to obtain 212 g of β-alanine with a content of 98.7%. The ammonia nitrogen content per gram of β-alanine was is 11 mg/g. The recovered liquid was 510 ml, and the ammonia nitrogen content in the recovered liquid was 2.5 g/L.
(2) The recovered liquid of the reaction solution A in the above (1) was added to the reaction solution B, concentrated under a reduced pressure to 1/5 of the volume of the original reaction solution volume, crystallized at a low temperature of 10°C, suction filtered and dried to obtain 393 g of β-alanine with a content of 98.3%. The ammonia nitrogen content per gram of β-alanine was 10 mg/g. The recovered liquid was 620 ml, and the ammonia nitrogen content in the recovered liquid was 5.7 g/L.
(3) The recovered liquid in the above (2) was added to the reaction solution C, concentrated under a reduced pressure to 1/5 of the volume of the original reaction solution volume, crystallized at a low temperature of 10°C, suction filtered and dried to obtain 410 g of β-alanine with a content of 98.1%. The ammonia nitrogen content per gram of β-alanine was 11 mg/g. The recovered liquid was 650 ml, and the ammonia nitrogen content in the recovered liquid was 5.8 g/L.
(4) The recovered liquid in the above (3) was directly concentrated under a reduced pressure to a solid to obtain 275 g of β-alanine with a content of 90.1%. The ammonia nitrogen content per gram of β-alanine was 11 mg/g.

It can be seen from the results of Example 3 and Example 4 that the β-alanine obtained by the salt-free process according to the present invention is significantly better than the β-alanine obtained by the salt process in terms of β-alanine content, ammonia nitrogen content, ammonia nitrogen content per gram of β-alanine and other aspects. The mother liquor obtained after crystallization in the salt-free process is still almost free of inorganic salts and other impurities upon repeated cycles, thereby reducing production costs.

### Example 5: Synthesis of Calcium β-Alanine

### Example 5.1

17.84 g (0.2 mole) of β-alanine obtained in Example 2 and 100 g of water were added into a three-necked flask successively and stirred at room temperature. After dissolved clarification, 6.21 g (0.108 mole) of 98% calcium oxide was added slowly, and then heated to reflux for 1 hour. The stirring was stopped, and the mixture was cooled naturally to 60°C, kept at the same temperature for 1 hour and filtered while the mixture is warm. The filtrate was added with the activated carbon, re-heated to 90°C and kept at the same temperature for 30 minutes. The mixture was filtered, and the filtrate was concentrated until a solid began to precipitate out. The isopropanol of a mass 4-6 times the mass of β-alanine was added, stirred and slurried to crystallize. The mixture was cooled to below 10°C, filtered, and dried to obtain 20.5 g of calcium β-alanine with a yield of 93%.

### Example 5.2

89.3 g (1 mole) of β-alanine obtained in Example 2 and 500 g of water were added into a three-necked flask successively and stirred at room temperature. After dissolved clarification, 30.98 g (0.54 mole) of 98% calcium oxide was added slowly, and then heated to reflux for 1 hour. The stirring was stopped, and the mixture was cooled naturally to 60°C, kept at the same temperature for 1 hour and filtered while the mixture is warm. The filtrate was added with the activated carbon, re-heated to 90°C and kept at the same temperature for 30 minutes. The mixture was filtered, and the filtrate was concentrated until a solid began to precipitate out. The isopropanol of a mass 4-6 times the mass of β-alanine was added, stirred and slurried to crystallize. The mixture was cooled to below 10°C, filtered, and dried to obtain 102.1 g of calcium β-alanine with a yield of 94.21%.

### Example 5.3

44.37 g (0.5 mole) of β-alanine obtained in Example 2 and 190 g of water were added into a three-necked flask successively and stirred at room temperature. After dissolved clarification, 15.45 g (0.27 mole) of 98% calcium oxide was added slowly, and then heated to reflux for 1 hour. The stirring was stopped, and the mixture was cooled naturally to 60°C, kept at the same temperature for 1 hour and filtered while the mixture is warm. The filtrate was added with the activated carbon, re-heated to 90°C and kept at the same temperature for 30 minutes. The mixture was filtered, and the filtrate was concentrated until a solid began to precipitate out. The ethanol of a mass 4-6 times the mass of β-alanine was added, stirred and slurried to crystallize. The mixture was cooled to below 10°C, filtered, and dried to obtain 43.6 g of calcium β-alanine with a yield of 80.96%.

### Example 5.4

195.8 g (2.2 moles) of β-alanine obtained in Example 2 and 1000 g of water were added into a three-necked flask successively and stirred at room temperature. After dissolved clarification, 67.9 g (1.19 moles) of 98% calcium oxide was added slowly, and then heated to reflux for 1 hour. The stirring was stopped, and the mixture was cooled naturally to 60°C, kept at the same temperature for 1 hour and filtered while the mixture is warm. The filtrate was added with the activated carbon, re-heated to 90°C and kept at the same temperature for 30 minutes. The mixture was filtered, and the filtrate was concentrated until a solid began to precipitate out. The mixture was cooled to below 10°C, filtered, and dried to obtain 116 g of calcium β-alanine with a yield of 75.50%.

### Example 5.5

89.1 g (1 mole) of β-alanine obtained in Example 2 and 160 g of water were added into a three-necked flask successively and stirred at room temperature. After dissolved clarification, 28.7 g (0.505 mole) of 98% calcium oxide was added slowly with a violent exotherm while the temperature was controlled below 60°C and then heated to reflux for another 0.5 h. The stirring was stopped, and the mixture was cooled naturally to 60°C, kept at the same temperature for 1 hour and filtered while the mixture is warm. The filtrate was added with the activated carbon, re-heated to 90°C and kept at the same temperature for 30 minutes. The mixture was filtered, and the mother liquor was concentrated and dried in an oven to obtain 104.8 g of calcium β-alanine with a yield of 97.01%.

### Example 5.6

89.1 g (1 mole) of β-alanine obtained in Example 2 and 160 g of water were added into a three-necked flask successively and stirred at room temperature. After dissolved clarification, 28.7 g (0.505 mole) of 98% calcium oxide was added slowly with a violent exotherm while the temperature was controlled below 60°C and then heated to reflux for 1 hour. The stirring was stopped, and the mixture was cooled naturally to 60°C, kept at the same temperature for 1 hour and filtered while the mixture is warm. The filtrate was added with the activated carbon, re-heated to 90°C and kept at the same temperature for 30 minutes. The mixture was filtered, and the mother liquor was concentrated and dried in an oven to obtain 105.5 g of calcium β-alanine with a yield of 97.71%.

### Example 5.7

89.1 g (1 mole) of β-alanine obtained in Example 2 and 160 g of water were added into a three-necked flask successively and stirred at room temperature. After dissolved clarification, 28.7 g (0.505 mole) of 98% calcium oxide was added slowly with a violent exotherm while the temperature was controlled below 60°C and then heated to reflux for another 3 hours. The stirring was stopped, and the mixture was cooled naturally to 60°C, kept at the same temperature for 1 hour and filtered while the mixture is warm. The filtrate was added with the activated carbon, re-heated to 90°C and kept at the same temperature for 30 minutes. The mixture was filtered, and the mother liquor was concentrated and dried in an oven to obtain 100.7 g of calcium β-alanine with a yield of 93.25%.

### Example 5.8

89.1 g (1 mole) of β-alanine obtained in Example 2 and 160 g of water were added into a three-necked flask successively and stirred at room temperature. After dissolved clarification, 28.2 g (0.5 mole) of 98% calcium oxide was added slowly with a violent exotherm while the temperature was controlled below 60°C and then heated to reflux for 1 hour. The stirring was stopped, and the mixture was cooled naturally to 60°C, kept at the same temperature for 1 hour and filtered while the mixture is warm. The filtrate was added with the activated carbon, re-heated to 90°C and kept at the same temperature for 30 minutes. The mixture was filtered, and the mother liquor was concentrated and dried in an oven to obtain 97.4 g of calcium β-alanine with a yield of 90.22%.

The reaction conditions and results of Examples 5.1 to 5.8 are summarized in Table 1 below.

**Table 1: Summary of Synthesis conditions and results of calcium β-alanine**

| **Example number** | **Feeding amount (weight ratio)** | **Dry weight (g)** | **Loss on drying** | **Yield** | **Content (based on calcium)** | **Content (based on β-alanine)** | **Note** |
|---|---|---|---|---|---|---|---|
| | **β-alanine: calcium oxide: water** | | | | | | |
| 5.1 | 17.84:6.21:100 | 20.5 | 1.9% | 93.00% | 99.80% | 99.10% | Crystallization with isopropanol |
| 5.2 | 89.3:30.98:500 | 102.1 | 0.7% | 94.21% | 98.76% | 97.38% | Scale up of crystallization with isopropanol |
| 5.3 | 44.37:15.45:190 | 43.6 | 1.6% | 80.96% | 96.60% | 99.96% | Crystallization with ethanol |
| 5.4 | 195.8:67.9:1000 | 116.0 | 2.3% | 75.50% | 95.68% | 103.17% | Crystallization with water |
| 5.5 | 89.1:28.7:160 | 104.8 | 1.0% | 97.01% | 99.81% | 102.05% | Reacting for 0.5 hour, and keeping at the same temperature for 1 hour |
| 5.6 | 89.1:28.7:160 | 105.5 | 1.1% | 97.71% | 99.51% | 101.17% | Reacting for 1 hour, and keeping at the same temperature for 1 hour |
| 5.7 | 89.1:28.7:160 | 100.7 | 0.9% | 93.25% | 98.73% | 102.85% | Reacting for 3 hours, and keeping at the same temperature for 1 hour |
| 5.8 | 89.1:28.2:160 | 97.4 | 1.3% | 90.22% | 92.60% | 104.90% | β-alanine: calcium oxide = 2:1 (molar ratio) |

### Example 6: Synthesis of calcium pantothenate

### Example 6.1

60.1 g (0.46 mole) of D-pantolactone and 225 g of methanol were added into a three-necked flask and stirred at room temperature. After dissolved clarification, 49.5 g (0.23 mole) of calcium β-alanine obtained in Example 5 was added slowly upon being stirred while agglomeration was prevented, and then the mixed was heated to 50°C. The reaction was carried out for 1 hour and then filtration was performed. The filtrate was stirred at 25°C for 2 hours and cooled to 0°C. A small amount of crystal water was added, then cooling was carried out to -5°C, crystallization was performed at -5°C for 16 hours, filtration was completed, and the filter cake was dried to obtain 57.1 g of white solid with a content of 99.7%, a yield of 53.1%, and a specific rotation of 26.1°.

### Example 6.2

58.5 g (0.45 mole) of D-pantolactone and 225 g of methanol were added into a three-necked flask and stirred at room temperature. After dissolved clarification, 49.5 g (0.23 mole) of calcium β-alanine obtained in Example 5 was added slowly upon being stirred while agglomeration was prevented, and then the mixed was heated to 50°C. The reaction was carried out for 1 hour and then filtration was performed. The filtrate was stirred at 25°C for 2 hours and cooled to 0°C. A small amount of crystal water was added (without adding seed crystal), then cooling was carried out to -10°C and crystallization was performed for 16 hours. A small amount of solid precipitated out, and filtration was carried out to obtain 18.1 g of white solid with a content of 98.0%, a yield of 16.4%, and a specific rotation of 25.2°.

### Example 6.3

585 g (4.5 moles) of D-pantolactone and 2250 g of methanol were added into a three-necked flask and stirred at room temperature. After dissolved clarification, 495 g (2.3 moles) of calcium β-alanine obtained in Example 5 was added slowly upon being stirred while agglomeration was prevented, and then the mixed was heated to 50°C. The reaction was carried out for 1 hour and then filtration was performed. The filtrate was stirred at 25°C for 2 hours and cooled to 0°C. A small amount of crystal water was added, then cooling was carried out to -5°C, crystallization was performed at -5°C for 16 hours, filtration was completed, and the filter cake was dried to obtain 566.7 g of white solid with a content of 99.6%, a yield of 52.8%, and a specific rotation of 26.4°.

### Example 6.4

317 g (2.44 moles) of D-pantolactone and 1442 g of methanol were added into a three-necked flask and stirred at room temperature. After dissolved clarification, 375 g (1.74 moles) of calcium β-alanine obtained in Example 5 was added slowly upon being stirred while agglomeration was prevented, and then the mixed was heated to 50°C. The reaction was carried out for 1 hour and then filtration was performed. The filtrate was stirred at 25°C for 2 hours and cooled to 0°C. A small amount of crystal water was added, then cooling was carried out to -10°C, crystallization was performed at -10°C for 16 hours, filtration was completed, and the filter cake was dried to obtain 529.9 g of white solid with a content of 99.8%, a yield of 91.1%, and a specific rotation of 26.7°.

### Example 6.5

58.5 g (0.45 mole) of D-pantolactone and 225 g of water were added into a three-necked flask and stirred at room temperature. After dissolved clarification, 49.5 g (0.23 mole) of calcium β-alanine obtained in Example 5 was added slowly upon being stirred while agglomeration was prevented, and then the mixed was heated to 50°C. The reaction was carried out for 1 hour, the reaction process was monitored by liquid phase, and the reaction yield calculated by the liquid phase normalization method was 2%-4%.

### Example 6.6

58.5 g (0.45 mole) of D-pantolactone and 225 g of methanol were added into a three-necked flask and stirred at room temperature. After dissolved clarification, 49.5 g (0.23 mole) of calcium β-alanine obtained in Example 5 was added slowly upon being stirred while agglomeration was prevented, and then the mixed was stirred at 25°C for 20 hours. The mixture was cooled to 0°C. A small amount of crystal water was added, then cooling was carried out to -10°C, crystallization was performed at -10°C for 16 hours, filtration was completed, and the filter cake was dried to obtain 92.3 g of white solid with a content of 98.1%, a yield of 83.6%, and a specific rotation of 26.3°.

### Example 6.7

58.5 g (0.45 mole) of D-pantolactone and 225 g of isopropanol were added into a three-necked flask and stirred at room temperature. After dissolved clarification, 49.5 g (0.23 mole) of calcium β-alanine obtained in Example 5 was added slowly upon being stirred while agglomeration was prevented, and then the mixed was heated to 50°C. The reaction was carried out for 1 hour, the reaction process was monitored by liquid phase, and the reaction yield calculated by the liquid phase normalization method was 5%-8%.

### Example 6.8

317 g (2.44 moles) of D-pantolactone and 1568 g of mother liquor of Example 6.4 were added into a three-necked flask and stirred at room temperature. After dissolved clarification, 375 g (1.74 moles) of calcium β-alanine obtained in Example 5 was added slowly upon being stirred while agglomeration was prevented, and then the mixed was heated to 50°C. The reaction was carried out for 1 hour and then filtration was performed. The filtrate was stirred at 25°C for 2 hours and cooled to 0°C. A small amount of crystal water was added, then cooling was carried out to -10°C, crystallization was performed at -10°C for 16 hours, and filtration was completed. The mother liquor and eluent were respectively used for the recycling of the next batch of reactions. The filter cake was dried to obtain 541 g of white solid with a content of 99.6%, a yield of 93.1%, and a specific rotation of 26.3°.

The reaction conditions and results of Examples 6.1 to 6.8 are summarized in Table 2 below.

**Table 2: Summary of synthesis conditions and results of calcium pantothenate**

| **Example number** | **Feeding ratio (mass ratio)** | **Loss on drying** | **Weigh t (g)** | **Specific rotatio n** | **Content** | **Reaction temperature (°C)** | **Reaction time (hours)** | **Crystallization temperature (°C)** | **Yield** |
|---|---|---|---|---|---|---|---|---|---|
| | **D-pantolactone : calcium β-alanine: solvent** | | | | | | | | |
| 6.1 | 60.1:49.5:225 (methanol) | 0.9% | 57.1 | 26.1° | 99.7% | 50, 25 | 1,2 | -5 | 53.1% |
| 6.2 | 58.5:49.5:225 (methanol) | \ | 18.1 | 25.2° | 98.0% | 50, 25 | 1,2 | -10 | 16.4% (without adding seed crystal) |
| 6.3 | 585:495:2250 (methanol) | 0.3% | 566.7 | 26.4° | 99.6% | 50, 25 | 1,2 | -5 | 52.8% |
| 6.4 | 317:375:1442 (methanol) | 0.6% | 529.9 | 26.7° | 99.8% | 50, 25 | 1,2 | -10 | 91.1% |
| 6.5 | 58.5:49.5:225 (water) | \ | \ | \ | \ | 80 | 1 | \ | 2%-4% |
| 6.6 | 58.5:49.5:225 (methanol) | 0.8% | 92.3 | 26.3° | 98.1% | 25 | 20 | -10 | 83.6% |
| 6.7 | 58.5:49.5:225 (isopropanol) | \ | \ | \ | \ | 80 | 1 | \ | 5%-8% |
| 6.8 | 317:375:1568 (mother liquor of Example 6.4) | 0.6% | 541 | 26.3° | 99.6% | 50, 25 | 1,2 | -10 | 93.1% |

### Example 7: Experiment for detecting the contents of chloride ions and sodium ions in calcium pantothenate

According to the European Pharmacopoeia, version 10.0, the content of chloride ions in calcium pantothenate shall not exceed 200 ppm. The contents of chloride ions and sodium ions in calcium pantothenate (hereinafter referred to as "testing sample 1") obtained by the method according to the present invention were detected and compared with the contents of chloride ions and sodium ions in two commercially available calcium pantothenate products by the applicant. The two commercially available calcium pantothenate products were purchased from Xinfa Pharmaceutical Co., Ltd. (hereinafter referred to as "testing sample 2") and Zhejiang Hangzhou Xinfu Pharmaceutical Co., Ltd. (hereinafter referred to as "testing sample 3"), respectively.

### Example 7.1 Investigation of linear relationship

The potassium chloride standard and sodium sulfate standard were obtained into an aqueous solution where the concentration of chloride ion is 4.79 mg/mL and the concentration of sodium ion is 3.25 mg/mL, and then the aqueous solution was diluted into aqueous solutions containing different concentrations of chloride and sodium ions. The concentrations of chloride ions were 0.0240 mg/mL, 0.0479 mg/mL, 0.0958 mg/mL, 0.1916 mg/mL, or 0.3832 mg/mL, and the concentrations of sodium ions were 0.0325 mg/mL, 0.0650 mg/mL, 0.13 mg/mL, 0.26 mg/mL, or 0.65 mg/mL. Each of the obtained solutions was injected with 10 µL, and the HPLC method was used for measurement. The HPLC parameters were as follows:
Chromatographic column: Acclaim Trinity P2, 3.0^{∗}100 mm^{∗}3 µm
Mobile phase: 100 mM ammonium formate buffer (pH 3.65), isocratic elution
Column flow rate: 0.3 mL/min
Column temperature: 30°C
Detector: ELSD

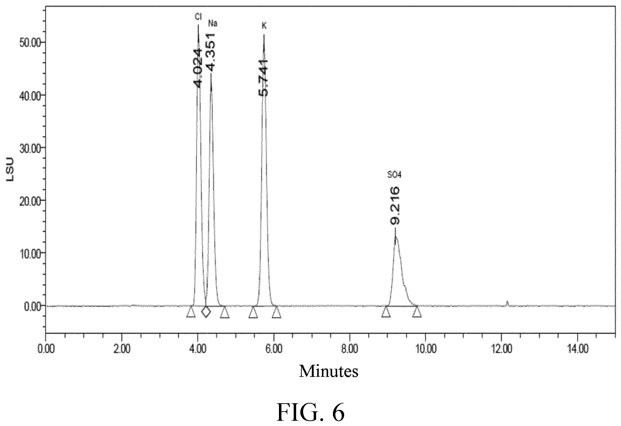

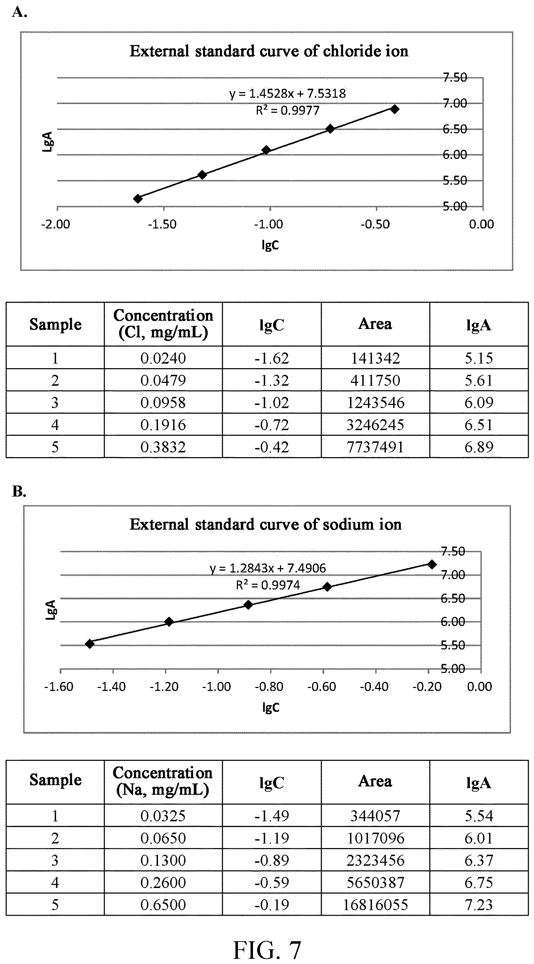

The HPLC chromatogram was shown in FIG. 6, and the chloride ion peaks at about 4.024 minutes. A standard curve was plotted with the logarithm of the injection concentration of chloride ion (LgC) versus the logarithm of the peak area (LgA), and the regression equation was calculated as Y=1.4528X+7.5318, R²=0.9977. See FIG. 7A for the standard curve. The results showed that when the injection concentration of chloride ion was in the range of 0.0240 mg/mL-0.3832 mg/mL, there was a good linear relationship with the peak area.

As shown in FIG. 6, the sodium ion peaked at about 4.351 minutes. A standard curve was plotted with the logarithm of the injection concentration of sodium ion (LgC) versus the logarithm of the peak area (LgA), and the regression equation was calculated as Y=1.2843X+7.4906, R²=0.9974. See FIG. 7B for the standard curve. The results showed that when the injection concentration of sodium ion was in the range of 0.0325 mg/mL-0.6500 mg/mL, there was a good linear relationship with the peak area.

### Example 7.2 Recovery rate of chloride ion

396.3 mg of calcium pantothenate testing sample 1 was weighed, dissolved and diluted to 10 mL by adding water, and the solution was used as a sample. 401.5 mg of calcium pantothenate testing sample 1 was weighed, 0.2 mL of chloride ion standard sample (4.79 mg/mL) was added, and the mixture was dissolved and diluted to 10 mL by adding water and used as a spiked sample. The content of chloride ion was calculated with the standard curve, and the recovery rate of chloride ion was 105.4%, indicating a good recovery rate.

### Example 7.3 Detection limit and quantification limit of chloride ion

392.6 mg of calcium pantothenate testing sample 1 was weighed, 0.2 mL of potassium chloride standard sample with a chloride ion at concentration of 0.0479 mg/mL was added, and the mixture was dissolved and diluted to 10 mL by adding water. HPLC was used for the determination.

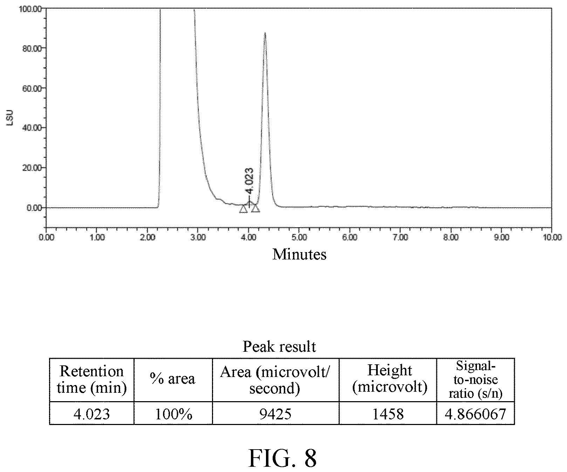

The results of the detection limit experiment were shown in FIG. 8, and the signal-to-noise ratio of chloride ions was greater than 3. The results showed that the detection limit of chloride ion in calcium pantothenate aqueous solution was 0.0025% (by weight).

400.2 mg of calcium pantothenate testing sample 1 was weighed, 1.0 mL of potassium chloride standard sample with a chloride ion at concentration of 0.0479 mg/mL was added, and the mixture was dissolved and diluted to 10 mL by adding water. HPLC was used for the determination.

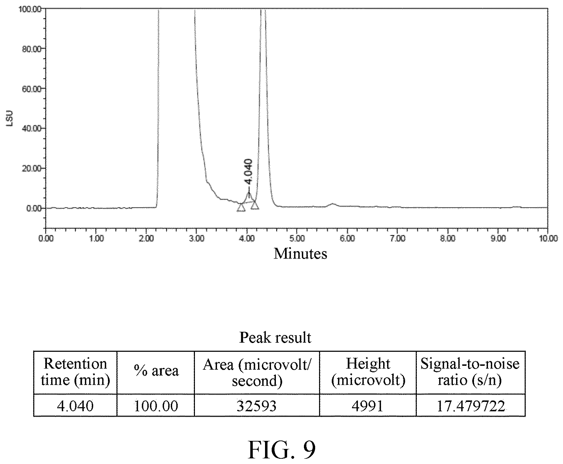

The results of the quantification limit experiment were shown in FIG. 9, and the signal-to-noise ratio of chloride ions was greater than 10. The results showed that the quantification limit of chloride ion in calcium pantothenate aqueous solution was 0.0125% (by weight).

### Example 7.4 Calcium pantothenate sample test

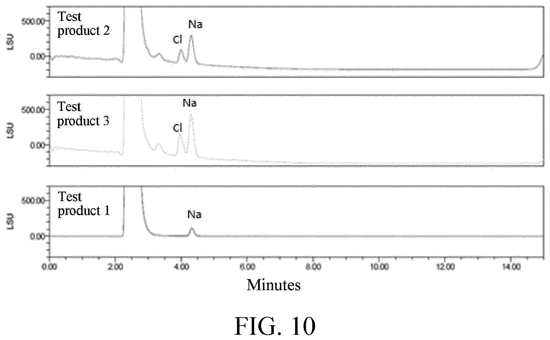

Testing sample 1, testing sample 2, and testing sample 3 of calcium pantothenate were respectively obtained into a 40 mg/mL calcium pantothenate aqueous solution for HPLC detection. The HPLC detection results were shown in Table 3 and FIG. 10. The results showed that no chloride ions were detected in testing sample 1, while chloride ions were detected in both testing sample 2 and testing sample 3. The sodium ion content (by weight) detected in testing sample 1 was 0.18%, and this was much lower than the sodium ion content in testing sample 2 and testing sample 3 (by weight, 0.50% and 0.68%, respectively).

**Table 3: Contents of chloride ion and sodium ion in each sample**

| **Sample** | **Chloride ion content** (by weight) | **Sodium ion content** (by weight) |
|---|---|---|
| Testing sample 1 | Not detected | 0.18% |
| Testing sample 2 | 0.12% | 0.50% |
| Testing sample 3 | 0.19% | 0.68% |

## Claims

1. A method for preparing β-alanine, comprising:
preparing a β-alanine product from a reactant containing fumaric acid and aqueous ammonia in the presence of a catalyst,
wherein the catalyst comprises a catalytic composition containing an aspartase and an L-aspartate-α-decarboxylase, and
adding fumaric acid during reaction, wherein total mole of the fumaric acid added is equal to an initial mole of the aqueous ammonia in the reactant minus an initial mole of the fumaric acid in the reactant.

2. The preparation method according to claim 1, wherein the catalytic composition contains a purified aspartase and a purified L-aspartate-α-decarboxylase.

3. The preparation method according to claim 1, wherein the catalytic composition contains bacteria expressing aspartase and L-aspartate-α-decarboxylase.

4. The preparation method according to claim 3, wherein the bacteria comprise wet bacteria, immobilized bacteria, or disrupted liquid of bacteria.

5. The preparation method according to claim 3, wherein the bacteria are derived from recombinantly engineered bacteria.

6. The preparation method according to any one of claims 3-5, wherein the bacteria comprise bacteria expressing aspartase alone and bacteria expressing L-aspartate-α-decarboxylase alone.

7. The preparation method according to claim 6, wherein a weight percentage of the bacteria expressing aspartase alone to the initial fumaric acid in the reactant is 0.5%-4% (w/w); and a weight percentage of the bacteria expressing L-aspartate-α-decarboxylase alone to the initial fumaric acid in the reactant is 10%-30% (w/w).

8. The preparation method according to any one of claims 3-5, wherein the bacteria comprise bacteria co-expressing aspartase and L-aspartate-α-decarboxylase.

9. The preparation method according to claim 8, wherein the weight percentage of the bacteria co-expressing aspartase and L-aspartate-α-decarboxylase to the initial fumaric acid in the reactant is 10%-40% (w/w).

10. The preparation method according to any one of claims 1-9, wherein the aspartase is derived from *Anoxybacillus flavithermus* or *Geobacillus thermodenitrificans;* and the L-aspartate-α-decarboxylase is derived from *Bacillus thermotolerans, Anoxybacillus flavithermus* or *Methanocaldococcus jannaschii.*

11. The preparation method according to any one of claims 1-10, wherein an initial molar ratio of the fumaric acid to the aqueous ammonia in the reactant is 1:2.

12. The preparation method according to any one of claims 1-11, wherein the added fumaric acid is added in a fed-batch manner during the reaction.

13. The preparation method according to claim 12, wherein the concentration of fumaric acid is ranged from 50 to 400 g/L, and a fed-batch speed thereof allows a pH value to be controlled at 6.8-7.2 during the reaction.

14. The preparation method according to any one of claims 1-13, wherein a reaction temperature is controlled at 25°C-55°C.

15. The preparation method according to any one of claims 1-14, further comprising removing residues in the catalytic composition after the catalytic reaction is completed.

16. The preparation method according to claim 15, further comprising crystallizing the β-alanine product.

17. The preparation method according to claim 16, wherein a mother liquor is obtained after the crystallization, and the content of inorganic salts in the mother liquor is less than 10 g/L.

18. The preparation method according to claim 16, wherein β-alanine crystal is obtained after the crystallization, and a content of inorganic salts in the crystal is less than 20 mg/g.

19. A method for preparing β-alanine salt, comprising the following steps:
(a) preparing β-alanine by the preparation method according to any one of claims 1-18; and
(b) reacting the β-alanine obtained in step (a) with an alkaline solution.

20. The preparation method according to claim 19, wherein the β-alanine salt is an alkali metal or alkaline earth metal salt of β-alanine, and the alkaline solution contains alkali metal or alkaline earth metal cations.

21. The preparation method according to claim 19 or 20, wherein the alkaline solution is selected from the group consisting of KOH, NaOH, Ca(OH)₂, Mg(OH)₂, Al(OH)₃, or a combination thereof.

22. The preparation method according to claim 21, wherein the Ca(OH)₂ is obtained from a reaction of calcium oxide with water.

23. The preparation method according to any one of claims 19-22, wherein the β-alanine salt is calcium β-alanine, sodium β-alanine, or potassium β-alanine.

24. The preparation method according to claim 23, wherein the β-alanine salt is calcium β-alanine.

25. The preparation method according to any one of claims 19-24, further comprising crystallizing the β-alanine salt.

26. A method for preparing pantothenate, comprising the following steps:
(a) preparing β-alanine by the method according to any one of claims 1-18;
(b) reacting the β-alanine obtained in step (a) with an alkaline solution to prepare β-alanine salt; and
(c) reacting pantolactone or pantoic acid with the β-alanine salt obtained in step (b).

27. The preparation method according to claim 26, wherein the pantolactone is D-pantolactone, and the pantoic acid is D-pantoic acid.

28. The preparation method according to claim 26 or 27, wherein the pantolactone or pantoic acid is dissolved in a solvent before reaction with the β-alanine salt obtained in step (b).

29. The preparation method according to claim 28, wherein the solvent is methanol or ethanol.

30. The preparation method according to any one of claims 26-29, wherein the pantothenate is calcium pantothenate, sodium pantothenate, or potassium pantothenate.

31. The preparation method according to claim 30, wherein the pantothenate is calcium pantothenate.

32. The preparation method according to any one of claims 26-30, further comprising crystallizing the pantothenate.

33. A calcium pantothenate, wherein content of a chloride ion (by weight) is not higher than 190 ppm, and/or content of a sodium ion (by weight) is not higher than 2200 ppm.

34. The calcium pantothenate according to claim 33, obtained by the method according to any one of claims 26-32.


Sequence Listing

<110>  Guang'an Mojia Biotech Co., Ltd.

<120>  Method for preparing β-alanine, β-alanine salt and pantothenate

<130>  065954-8002EP01

<160>  5

<170>  PatentIn version 3.5

<210>  1
<211>  1410
<212>  DNA
<213>  Anoxybacillus flavithermus WK1

<400>  1
ttatttccct gcaatgcccg gacttgtcat cgcatacggg tctaaaatat catgtaattg
60

cttttcattc aacaaatcgt actccaagca tagctcgcgc actgattttc ccgtatgaag
120

cgcttctttc gcgatgcgcg atgccgcttc ataaccgatg tacggattga cagctgtaat
180

gacgccgatg ctattttcga cgtgttgctt cattcgctct tcgttcgctt caatgccagc
240

taaacaatag tctgtaaaca cacgaaagac gttgtccatc atatgaagcg actgaagtaa
300

gttaaacaca agcaccggct ccatgacgtt tagctctagc tgtcctgctt ctgacgctaa
360

gcaaatcgtt tggtcattac cgatcacttg aaacgccact tggttgacga cttccgccat
420

aaccggattc actttccccg gcataatcga cgatcccggc tgacgagcag gcaacgtaat
480

ttctccaagc cctgcccgcg gaccagacgc catgagacga agatcgtttg cgattttcga
540

catgttgatc atacatattt ttaaggcagc tgatacttct gtatacgcat ctgtattttg
600

tgtcgcatca acgagatgtt ctgcgcgcac aagcggcaat tcggtcaagc gaacgagatg
660

aaaaatgacg cgttcaatat agaccggatc ggcatttaat cccgttccga ccgccgttgc
720

accaatattg acttcgtaca aatgttggcg cgaatgagca atgcgctcca tatcgcgctt
780

gacgacgcga cgatacgctt caaattcttg tccgagccga atcggaacgg catcttgtaa
840

atgcgtccgt cccatcttaa tgacatgatc aaactgtttc gctttatcgg cgaacacatc
900

gtgcatatgt ttcatcgtct gaagtaattg gttcaccatc tttaacactg caatatgaat
960

cgctgtcggg aaaacgtcgt ttgtcgattg cgacatgttg acgtgcgtat ttgggctaat
1020

gatatgataa tttccttttc gctctcctaa ccattcgagt gcgcgattcg cgatcacttc
1080

gttcgtgttc atattgatcg acgttcctgc gccaccttga atcgggtcga cgataaattg
1140

atcgtgccat tgtcctgcga tcacttcatc tgccgctttt acgatcgctt caccgatgcg
1200

acgatcaagt tgccccgttt ccatattcgc aagcgccgcc gcttttttca cgatcgccat
1260

cgcttgaatg agttcgcgat gaagacgata cccggtaatc gggaagtttt ctgcggcgcg
1320

catcgtttga atgccgtaat acgcatcgta tggaatttct ttttcgccaa gcaaatctcg
1380

ttcaagtcgt actgcttttg tttctagcat
1410



<210>  2
<211>  1437
<212>  DNA
<213>  Geobacillus thermodenitrificans NG80-2

<400>  2
ttagggcttg tcaaaagaaa taacactgag gatgcctgga ctcgtcattt tataaggatc
60

taaaatatga tccaattctt tttccgttaa caaaccgtat tgcaaacata gtttacggat
120

ggattcccct tctaagatcg cctgttgagc aatacgtgcg gccgtttcat atccgatata
180

tgggctaaca gctgtaacca cactgacact tttttctaca taatgtctca aacgttcctc
240

gttggcttca atacccttta aacaataatt tgtaaacaca tgaaacgcat tcgtcattat
300

cgtaatcgat tgaagcagat taaagacgag cacaggttcc attacattta gttccagttg
360

acctgcctca gaagcaagac aaatcgtatg gtcattgcct atcacttgga aagcgacttg
420

gttaatcacc tcggccataa caggattaac tttacctggc ataatagacg agccaggctg
480

acggggcggc aatgtaattt ctcccaaacc ggctctaggt cctgacgcca ttaaacgcaa
540

gtcattggct attttagaca tattgatcat acacactttt agagcggacg atacttctgt
600

atatgcatcc gtattttgtg ttgcatccac caaatgttca gctctaacta atggcaaccc
660

gctaatttct gataaatatt tgacaacacc ctcaatatat cggggatcag catttaaccc
720

cgtcccgact gcggtggctc ccatattgat ttcatataaa tattgacggg agcgatcgat
780

gcgttggata tcacgctcta acacgcagct atatgcttca aactcttgcc ctaaacgaat
840

gggtacagca tcttgaagat gggtgcggcc tattttaatg acatggtcaa attgccgggc
900

tttttccttc atcactgcat gcatatatcg catcgtatgg agcagtttgt ctagtaagtt
960

tagtaaggca atatgaaccg ctgttgggaa aacatcgttg gttgactgag acatattcac
1020

atgattattt ggacttaaat aaccatattc tcctttttca tatccaagca attcgagagc
1080

acgattggcg atgacttcat ttgcattcat atttatcgat gtacccgctc cgccttgtat
1140

aggatcaacg ataaagtgct catgccattt tcctgcaatg atttcatctg cagctttaac
1200

gatggcctcg cctatttcag cttttaatcg ttttgtgtcc atattggcta aggcagctgc
1260

tttcttcacc atcgccaaag ctttaatcaa ttccttgtga attcgatatc cggtgatagg
1320

aaagttctct agggctctta atgtttgtat cccataataa gcatctatcg gaacttcttt
1380

atcccctagg aaatcccttt caatccgaac cttactattg gtattagcgg ttgccat
1437

<210> 3
<211> 384
<212> DNA

<213>  Bacillus thermotolerans

<400>  3
atgtttcgta ctatgatgaa cgggaaaatt caccgtgcga ctgtcacgga agctaactta
60

aactatgtag gcagtatcac gattgaccaa gatctgctgg atgcggcagg catggttgaa
120

aatgaaaaag tgcaaattgt caacaataac aatggcgccc gtctggaaac atacattatc
180

tcaggtgagc ggggcagcgg tgtagtttgc ttaaacggag cagcagcccg tctcgttcag
240

cctggagata aggttattat catttcgtat gtcatggtgg ctgaagagca agtgaaggat
300

catcagccaa agattgtcgt agcagatgaa ttaaaccgga ttgatcagct gcttcatcag
360

gaaatagcgg gcacagtttt ataa
384


<210>  4
<211>  384
<212>  DNA
<213>  Anoxybacillus flavithermus AK1

<400>  4
atgtttcgta cattaatgaa tgctaaaatt catcgtgctc gcgtgacgga agcgaattta
60

aactatgtcg gcagcattac gatcgatgaa gatattttag atgcggtcgg catggtgcca
120

aatgaaaaag tacaaattgt aaacaataac aatggggcac gatttgaaac atatattatt
180

ccaggtgaaa gaggaagcgg cgtattttgt ttgaacgggg cagccgctcg cctcgttcaa
240

aaagacgata ttattatcgt tatttcgtac gtgctcgtac cagaagaaaa attagcttcg
300

caccgtccaa aaattgcaat tatggacgag cataatcgga ttaaagaatt gatcgtgcaa
360

gagccggcgg cgaccgtttt ataa
384


<210>  5
<211>  1182
<212>  DNA
<213>  Methanocaldococcus jannaschii

<400> 5
atgcaggaaa aaggtgttag tgagaaagaa attttagagg aattgaaaaa atataggagt
60

ttggatttaa agtatgaaga tggaaatatt ttcggttcaa tgtgttccaa tgtattacca
120

ataacaagaa aaattgtaga tatcttctta gaaacaaact tgggagaccc tggactattt
180

aaagggacta aattgttaga agaaaaagct gtggctttat tgggttcttt gttaaataac
240

aaagatgcct atggacatat agttagtgga gggactgaag ccaacttaat ggctttaaga
300

tgcataaaaa atatatggag ggaaaaaagg agaaagggct tatcaaaaaa tgaacatcca
360

aagattatcg ttccaataac tgcccatttc tcattcgaaa aaggaagaga aatgatggac
420

ttagagtata tctatgcccc aattaaagaa gattatacaa tagatgagaa attcgttaaa
480

gatgccgtag aggattatga tgtagatggc attataggaa ttgctggaac aacagagctt
540

ggaactattg acaacataga ggagctaagt aaaatagcaa aagaaaacaa catttatatc
600

catgtagatg cggcatttgg aggcttagta attccatttt tagatgataa atataagaaa
660

aaaggagtaa attataaatt tgacttttct ttgggagttg attctataac catagacccc
720

cataaaatgg ggcactgccc aatcccaagt ggagggattc tatttaaaga tataggttat
780

aaaagatatt tggatgttga tgccccttat ttaactgaaa caagacaggc aacaatctta
840

ggaacaaggg ttggattcgg aggagcctgc acttatgcag ttttaagata tttaggtaga
900

gagggacagc gaaaaattgt taatgaatgt atggaaaaca ccctttatct ttacaaaaaa
960

ttgaaggaaa ataattttaa accagtcatt gaaccaatat taaatattgt tgcaattgaa
1020

gatgaagatt ataaagaagt ctgcaaaaaa cttagagata gaggcattta cgtttcagtt
1080

tgcaattgtg ttaaagcttt gagaatcgtt gttatgccac atattaagag ggagcatata
1140

gataatttta tcgaaatatt gaatagtatt aaaaaggatt ga
1182